# EUROPÄISCHE PATENTSCHRIFT

(11) **EP 3 344 781 B1**
(45) Veröffentlichungstag und Bekanntmachung des Hinweises auf die Patenterteilung: **18.12.2019**
(21) Anmeldenummer: 16770894.0
(22) Anmeldetag: 01.09.2016
(51) Int. Cl.: C12Q 1/6883

(54) **VERWENDUNG VON IM BLUTSERUM ODER BLUTPLASMA ZIRKULIERENDEN MICRORNAS ZUR IDENTIFIKATION BIOPSIEPFLICHTIGER PATIENTEN UND ALS MARKER ZUR DIFFERENTIALDIAGNOSE EINZELNER NICHT-ISCHÄMISCHER KARDIOMYOPATHIEN ODER SPEICHERERKRANKUNGEN DES HERZENS**
USE OF IN BLOOD SERA OR BLOOD PLASMA CIRCULATING MICRORNAS FOR IDENTIFICATION OF PATIENTS REQUIREING BIOPSIE AND AS MARKER FOR THE DIFFERENTIAL DIAGNOSIS OF SPECIFIC NON-ISCHEMIC CARDIOMYOPATHIES OR STORAGE DISEASE IN THE HEART
UTILISATION DE MIRNA CIRCULANT DU SERUM OU DU PLASMA SANGUIN POUR IDENTIFIER LES PATIENTS REQUERANTS UNE BIOPSIE ET COMME MARQUEUR POUR LE DIAGNOSITC DIFFERENTIEL DE CARDIOMYOPATHIES NON ISCHEMIC SPECIFIC DE MALADIE DU STOCKAGE DU COEUR

(30) Priorität: 02.09.2015 DE 102015216782
(43) Veröffentlichungstag der Anmeldung: 11.07.2018
(73) Patentinhaber: IKDT Institut Kardiale Diagnostik und Therapie GmbH, 12203 Berlin (DE)
(72) Erfinder: SCHULTHEISS, Heinz-Peter, 14163 Berlin (DE); LASSNER, Dirk, 14532 Stahnsdorf (DE); ROHDE, Maria, 10115 Berlin (DE); SIEGISMUND, Christine, 10367 Berlin (DE); KOHLENBERG, Max, 12555 Berlin (DE)
(74) Vertreter: Maikowski & Ninnemann Patentanwälte Partnerschaft mbB
(86) Internationale Anmeldenummer: PCT/EP2016/070643
(87) Internationale Veröffentlichungsnummer: WO 2017/037190

(56) Entgegenhaltungen:
- EP-A1- 2 354 246
- WO-A1-2013/093870
- DE-A1-102012 101 557
- SIEGISMUND CHRISTINE S ET AL: "Multiparametric diagnostics of cardiomyopathies by microRNA signatures", MIKROCHIMICA ACTA, SPRINGER VERLAG, VIENNA, AT, Bd. 181, Nr. 13, 8. April 2014 (2014-04-08), Seiten 1647-1653, XP035392222, ISSN: 0026-3672, DOI: 10.1007/S00604-014-1249-Y [gefunden am 2014-04-08]
- MENG XUN ET AL: "Differential expression of miRNAs in enterovirus 71-infected cells", VIROLOGY JOURNAL, BIOMED CENTRAL, LONDON, GB, Bd. 12, Nr. 1, 10. April 2015 (2015-04-10) , Seite 56, XP021221553, ISSN: 1743-422X, DOI: 10.1186/S12985-015-0288-2
- DIRK LASSNER ET AL: "Recent Advances in Molecular Diagnostics and Treatment of Heart Muscle Diseases", JOURNAL OF ANALYTICAL SCIENCES, METHODS AND INSTRUMENTATION, Bd. 03, Nr. 02, 1. Januar 2013 (2013-01-01), Seiten 98-109, XP055329100, ISSN: 2164-2745, DOI: 10.4236/jasmi.2013.32012
- DIRK LASSNER ET AL: "Myocarditis-Personalized Medicine by Expanded Endomyocardial Biopsy Diagnostics", WORLD JOURNAL OF CARDIOVASCULAR DISEASES, Bd. 04, Nr. 06, 1. Januar 2014 (2014-01-01), Seiten 325-340, XP055329101, ISSN: 2164-5329, DOI: 10.4236/wjcd.2014.46042
- P VAN DEN HOOGEN ET AL: "Send Orders for Reprints to reprints@benthamscience.ae Heart Failure in Chronic Myocarditis: A Role for microRNAs?", CURRENT GENOMICS, Bd. 16, 1. April 2015 (2015-04-01), Seiten 88-94, XP055329105,

### SEQUENCE LISTING

<110> IKDT Institut kardiale Diagnostik und Therapie GmbH
<120> Verwendung von im Blutserum oder Blutplasma zirkulierenden microRNAs zur Identifikation biopsiepflichtiger Patienten und als Marker zur Differentialdiagnose einzelner nicht-ischämischer Kardiomyopathien oder Speichererkrankungen des Herzens
<130> IKD102WO
<150> DE 10 2015 216 782.8
   <151> 2015-09-02
<160> 175
<170> PatentIn version 3.5
<210> 1
   <211> 23
   <212> RNA
   <213> Homo sapiens
<400> 1
   uacccuguag auccgaauuu gug 23
<210> 2
   <211> 23
   <212> RNA
   <213> Homo sapiens
<400> 2
   uacccuguag aaccgaauuu gug 23
<210> 3
   <211> 22
   <212> RNA
   <213> Homo sapiens
<400> 3
   cagugcaaug augaaagggc au 22
<210> 4
   <211> 22
   <212> RNA
   <213> Homo sapiens
<400> 4
   ucagugcauc acagaacuuu gu 22
<210> 5
   <211> 21
   <212> RNA
   <213> Homo sapiens
<400> 5
   ucagugcaug acagaacuug g 21
<210> 6
   <211> 22
   <212> RNA
   <213> Homo sapiens
<400> 6
   uggagagaaa ggcaguuccu ga 22
<210> 7
   <211> 21
   <212> RNA
   <213> Homo sapiens
<400> 7
   uucacagugg cuaaguucug c 21
<210> 8
   <211> 22
   <212> RNA
   <213> Homo sapiens
<400> 8
   cuuucagucg gauguuugca gc 22
<210> 9
   <211> 23
   <212> RNA
   <213> Homo sapiens
<400> 9
   ucaagagcaa uaacgaaaaa ugu 23
<210> 10
   <211> 22
   <212> RNA
   <213> Homo sapiens
<400> 10
   ugagguagua gauuguauag uu 22
<210> 11
   <211> 23
   <212> RNA
   <213> Homo sapiens
<400> 11
   agcagcauug uacagggcua uga 23
<210> 12
   <211> 22
   <212> RNA
   <213> Homo sapiens
<400> 12
   uucaccaccu ucuccaccca gc 22
<210> 13
   <211> 21
   <212> RNA
   <213> Homo sapiens
<400> 13
   augaccuaug aauugacaga c 21
<210> 14
   <211> 22
   <212> RNA
   <213> Homo sapiens
<400> 14
   ugucaguuug ucaaauaccc ca 22
<210> 15
   <211> 21
   <212> RNA
   <213> Homo sapiens
<400> 15
   aucacauugc cagggauuuc c 21
<210> 16
   <211> 23
   <212> RNA
   <213> Homo sapiens
<400> 16
   gcaaagcaca cggccugcag aga 23
<210> 17
   <211> 21
   <212> RNA
   <213> Homo sapiens
<400> 17
   gaacggcuuc auacaggagu u 21
<210> 18
   <211> 23
   <212> RNA
   <213> Homo sapiens
<400> 18
   ugagcgccuc gacgacagag ccg 23
<210> 19
   <211> 23
   <212> RNA
   <213> Homo sapiens
<400> 19
   ucccuguccu ccaggagcuc acg 23
<210> 20
   <211> 21
   <212> RNA
   <213> Homo sapiens
<400> 20
   uaguagaccg uauagcguac g 21
<210> 21
   <211> 23
   <212> RNA
   <213> Homo sapiens
<400> 21
   uagugcaaua uugcuuauag ggu 23
<210> 22
   <211> 22
   <212> RNA
   <213> Homo sapiens
<400> 22
   gucauacacg gcucuccucu cu 22
<210> 23
   <211> 22
   <212> RNA
   <213> Homo sapiens
<400> 23
   aaucguacag ggucauccac uu 22
<210> 24
   <211> 22
   <212> RNA
   <213> Homo sapiens
<400> 24
   ugaaacauac acgggaaacc uc 22
<210> 25
   <211> 22
   <212> RNA
   <213> Homo sapiens
<400> 25
   cgucaacacu ugcugguuuc cu 22
<210> 26
   <211> 22
   <212> RNA
   <213> Homo sapiens
<400> 26
   augcugacau auuuacuaga gg 22
<210> 27
   <211> 21
   <212> RNA
   <213> Homo sapiens
<400> 27
   uccgguucuc agggcuccac c 21
<210> 28
   <211> 22
   <212> RNA
   <213> Homo sapiens
<400> 28
   ugcggggcua gggcuaacag ca 22
<210> 29
   <211> 21
   <212> RNA
   <213> Homo sapiens
<400> 29
   uuaauaucgg acaaccauug u 21
<210> 30
   <211> 22
   <212> RNA
   <213> Homo sapiens
<400> 30
   aaacaaacau ggugcacuuc uu 22
<210> 31
   <211> 17
   <212> RNA
   <213> Homo sapiens
<400> 31
   ucccuguucg ggcgcca 17
<210> 32
   <211> 22
   <212> RNA
   <213> Homo sapiens
<400> 32
   caucaucguc ucaaaugagu cu 22
<210> 33
   <211> 22
   <212> RNA
   <213> Homo sapiens
<400> 33
   ucgugucuug uguugcagcc gg 22
<210> 34
   <211> 23
   <212> RNA
   <213> Homo sapiens
<400> 34
   cccaguguuc agacuaccug uuc 23
<210> 35
   <211> 21
   <212> RNA
   <213> Homo sapiens
<400> 35
   augaccuaug aauugacaga c 21
<210> 36
   <211> 22
   <212> RNA
   <213> Homo sapiens
<400> 36
   acuggacuua gggucagaag gc 22
<210> 37
   <211> 22
   <212> RNA
   <213> Homo sapiens
<400> 37
   cgucccgggg cugcgcgagg ca 22
<210> 38
   <211> 21
   <212> RNA
   <213> Homo sapiens
<400> 38
   cugggcucgg gacgcgcggc u 21
<210> 39
   <211> 21
   <212> RNA
   <213> Homo sapiens
<400> 39
   guuucaccau guuggucagg c 21
<210> 40
   <211> 22
   <212> RNA
   <213> Homo sapiens
<400> 40
   caaaaaucuc aauuacuuuu gc 22
<210> 41
   <211> 22
   <212> RNA
   <213> Homo sapiens
<400> 41
   aaaaguaauu gcgguuuuug cc 22
<210> 42
   <211> 21
   <212> RNA
   <213> Homo sapiens
<400> 42
   aggaggcagc gcucucagga c 21
<210> 43
   <211> 22
   <212> RNA
   <213> Homo sapiens
<400> 43
   uuuccggcuc gcgugggugu gu 22
<210> 44
   <211> 22
   <212> RNA
   <213> Homo sapiens
<400> 44
   uggaauguaa agaaguaugu au 22
<210> 45
   <211> 22
   <212> RNA
   <213> Homo sapiens
<400> 45
   cagugguuuu acccuauggu ag 22
<210> 46
   <211> 22
   <212> RNA
   <213> Homo sapiens
<400> 46
   cugguacagg ccugggggac ag 22
<210> 47
   <211> 22
   <212> RNA
   <213> Homo sapiens
<400> 47
   uagcagcacg uaaauauugg cg 22
<210> 48
   <211> 21
   <212> RNA
   <213> Homo sapiens
<400> 48
   augaccuaug aauugacaga c 21
<210> 49
   <211> 22
   <212> RNA
   <213> Homo sapiens
<400> 49
   cauugcacuu gucucggucu ga 22
<210> 50
   <211> 22
   <212> RNA
   <213> Homo sapiens
<400> 50
   uguaaacauc cucgacugga ag 22
<210> 51
   <211> 22
   <212> RNA
   <213> Homo sapiens
<400> 51
   uguaaacauc cuugacugga ag 22
<210> 52
   <211> 24
   <212> RNA
   <213> Homo sapiens
<400> 52
   uguggacagu gagguagagg gagu 24
<210> 53
   <211> 20
   <212> RNA
   <213> Homo sapiens
<400> 53
   aggcauggga ggucagguga 20
<210> 54
   <211> 23
   <212> RNA
   <213> Homo sapiens
<400> 54
   ucaaggccag aggucccaca gca 23
<210> 55
   <211> 22
   <212> RNA
   <213> Homo sapiens
<400> 55
   acuggacuua gggucagaag gc 22
<210> 56
   <211> 18
   <212> RNA
   <213> Homo sapiens
<400> 56
   cagcaguccc ucccccug 18
<210> 57
   <211> 17
   <212> RNA
   <213> Homo sapiens
<400> 57
   uucugagcug aggacag 17
<210> 58
   <211> 18
   <212> RNA
   <213> Homo sapiens
<400> 58
   gaggcugaag gaagaugg 18
<210> 59
   <211> 20
   <212> RNA
   <213> Homo sapiens
<400> 59
   aaaagcuggg cugagaggcg 20
<210> 60
   <211> 22
   <212> RNA
   <213> Homo sapiens
<400> 60
   uggcggcggu aguuaugggc uu 22
<210> 61
   <211> 20
   <212> RNA
   <213> Homo sapiens
<400> 61
   cggggugggu gaggucgggc 20
<210> 62
   <211> 22
   <212> RNA
   <213> Homo sapiens
<400> 62
   uccauguuuc cuucccccuu cu 22
<210> 63
   <211> 22
   <212> RNA
   <213> Homo sapiens
<400> 63
   aaaaguaauu gugguuuugg cc 22
<210> 64
   <211> 22
   <212> RNA
   <213> Homo sapiens
<400> 64
   caaaaaucuc aauuacuuuu gc 22
<210> 65
   <211> 22
   <212> RNA
   <213> Homo sapiens
<400> 65
   ugugucacuc gaugaccacu gu 22
<210> 66
   <211> 22
   <212> RNA
   <213> Homo sapiens
<400> 66
   uggucuagga uuguuggagg ag 22
<210> 67
   <211> 22
   <212> RNA
   <213> Homo sapiens
<400> 67
   guucucccaa cguaagccca gc 22
<210> 68
   <211> 22
   <212> RNA
   <213> Homo sapiens
<400> 68
   uacccauugc auaucggagu ug 22
<210> 69
   <211> 21
   <212> RNA
   <213> Homo sapiens
<400> 69
   cgcgggugcu uacugacccu u 21
<210> 70
   <211> 21
   <212> RNA
   <213> Homo sapiens
<400> 70
   gucagcggag gaaaagaaac u 21
<210> 71
   <211> 22
   <212> RNA
   <213> Homo sapiens
<400> 71
   auauaauaca accugcuaag ug 22
<210> 72
   <211> 23
   <212> RNA
   <213> Homo sapiens
<400> 72
   agcagcauug uacagggcua uga 23
<210> 73
   <211> 21
   <212> RNA
   <213> Homo sapiens
<400> 73
   uaaagugcug acagugcaga u 21
<210> 74
   <211> 22
   <212> RNA
   <213> Homo sapiens
<400> 74
   ucguaccgug aguaauaaug cg 22
<210> 75
   <211> 21
   <212> RNA
   <213> Homo sapiens
<400> 75
   cauuauuacu uuugguacgc g 21
<210> 76
   <211> 18
   <212> RNA
   <213> Homo sapiens
<400> 76
   gucccuguuc aggcgcca 18
<210> 77
   <211> 17
   <212> RNA
   <213> Homo sapiens
<400> 77
   ucccuguucg ggcgcca 17
<210> 78
   <211> 22
   <212> RNA
   <213> Homo sapiens
<400> 78
   uaacagucua cagccauggu cg 22
<210> 79
   <211> 21
   <212> RNA
   <213> Homo sapiens
<400> 79
   uaccacaggg uagaaccacg g 21
<210> 80
   <211> 23
   <212> RNA
   <213> Homo sapiens
<400> 80
   uguaguguuu ccuacuuuau gga 23
<210> 81
   <211> 22
   <212> RNA
   <213> Homo sapiens
<400> 81
   ugagaacuga auuccauagg cu 22
<210> 82
   <211> 22
   <212> RNA
   <213> Homo sapiens
<400> 82
   ucucccaacc cuuguaccag ug 22
<210> 83
   <211> 22
   <212> RNA
   <213> Homo sapiens
<400> 83
   uagcagcaca ucaugguuua ca 22
<210> 84
   <211> 22
   <212> RNA
   <213> Homo sapiens
<400> 84
   aacauucauu gcugucggug gg 22
<210> 85
   <211> 22
   <212> RNA
   <213> Homo sapiens
<400> 85
   caaagaauuc uccuuuuggg cu 22
<210> 86
   <211> 22
   <212> RNA
   <213> Homo sapiens
<400> 86
   uagcuuauca gacugauguu ga 22
<210> 87
   <211> 21
   <212> RNA
   <213> Homo sapiens
<400> 87
   agcuacaucu ggcuacuggg u 21
<210> 88
   <211> 22
   <212> RNA
   <213> Homo sapiens
<400> 88
   cguguauuug acaagcugag uu 22
<210> 89
   <211> 22
   <212> RNA
   <213> Homo sapiens
<400> 89
   uggcucaguu cagcaggaac ag 22
<210> 90
   <211> 21
   <212> RNA
   <213> Homo sapiens
<400> 90
   uucaaguaau ucaggauagg u 21
<210> 91
   <211> 22
   <212> RNA
   <213> Homo sapiens
<400> 91
   cuuucagucg gauguuugca gc 22
<210> 92
   <211> 22
   <212> RNA
   <213> Homo sapiens
<400> 92
   uguaaacauc cccgacugga ag 22
<210> 93
   <211> 22
   <212> RNA
   <213> Homo sapiens
<400> 93
   aaaagcuggg uugagagggc ga 22
<210> 94
   <211> 20
   <212> RNA
   <213> Homo sapiens
<400> 94
   aaaagcuggg uugagagggu 20
<210> 95
   <211> 22
   <212> RNA
   <213> Homo sapiens
<400> 95
   cuggcccucu cugcccuucc gu 22
<210> 96
   <211> 22
   <212> RNA
   <213> Homo sapiens
<400> 96
   uuuguucguu cggcucgcgu ga 22
<210> 97
   <211> 22
   <212> RNA
   <213> Homo sapiens
<400> 97
   cuccugacuc cagguccugu gu 22
<210> 98
   <211> 22
   <212> RNA
   <213> Homo sapiens
<400> 98
   agcucggucu gaggccccuc ag 22
<210> 99
   <211> 17
   <212> RNA
   <213> Homo sapiens
<400> 99
   accccacucc ugguacc 17
<210> 100
   <211> 22
   <212> RNA
   <213> Homo sapiens
<400> 100
   aaaccguuac cauuacugag uu 22
<210> 101
   <211> 17
   <212> RNA
   <213> Homo sapiens
<400> 101
   guggaccugg cugggac 17
<210> 102
   <211> 22
   <212> RNA
   <213> Homo sapiens
<400> 102
   aagacgggag gaaagaaggg ag 22
<210> 103
   <211> 21
   <212> RNA
   <213> Homo sapiens
<400> 103
   caaagcgcuu cccuuuggag c 21
<210> 104
   <211> 22
   <212> RNA
   <213> Homo sapiens
<400> 104
   caugccuuga guguaggacc gu 22
<210> 105
   <211> 22
   <212> RNA
   <213> Homo sapiens
<400> 105
   gagcuuauuc auaaaagugc ag 22
<210> 106
   <211> 22
   <212> RNA
   <213> Homo sapiens
<400> 106
   uggucuagga uuguuggagg ag 22
<210> 107
   <211> 22
   <212> RNA
   <213> Homo sapiens
<400> 107
   uacccauugc auaucggagu ug 22
<210> 108
   <211> 22
   <212> RNA
   <213> Homo sapiens
<400> 108
   uccauuacac uacccugccu cu 22
<210> 109
   <211> 22
   <212> RNA
   <213> Homo sapiens
<400> 109
   uauugcacuu gucccggccu gu 22
<210> 110
   <211> 22
   <212> RNA
   <213> Homo sapiens
<400> 110
   cacccguaga accgaccuug cg 22
<210> 111
   <211> 22
   <212> RNA
   <213> Homo sapiens
<400> 111
   ggagacgcgg cccuguugga gu 22
<210> 112
   <211> 22
   <212> RNA
   <213> Homo sapiens
<400> 112
   cugugcgugu gacagcggcu ga 22
<210> 113
   <211> 21
   <212> RNA
   <213> Homo sapiens
<400> 113
   agggcccccc cucaauccug u 21
<210> 114
   <211> 21
   <212> RNA
   <213> Homo sapiens
<400> 114
   cggggcagcu caguacagga u 21
<210> 115
   <211> 23
   <212> RNA
   <213> Homo sapiens
<400> 115
   uaauccuugc uaccugggug aga 23
<210> 116
   <211> 22
   <212> RNA
   <213> Homo sapiens
<400> 116
   ugugacagau ugauaacuga aa 22
<210> 117
   <211> 23
   <212> RNA
   <213> Homo sapiens
<400> 117
   ucauagcccu guacaaugcu gcu 23
<210> 118
   <211> 22
   <212> RNA
   <213> Homo sapiens
<400> 118
   ucccugagac ccuuuaaccu gu 22
<210> 119
   <211> 22
   <212> RNA
   <213> Homo sapiens
<400> 119
   cuuggcaccu agcaagcacu ca 22
<210> 120
   <211> 17
   <212> RNA
   <213> Homo sapiens
<400> 120
   ucccuguucg ggcgcca 17
<210> 121
   <211> 22
   <212> RNA
   <213> Homo sapiens
<400> 121
   gcuacuucac aacaccaggg cc 22
<210> 122
   <211> 22
   <212> RNA
   <213> Homo sapiens
<400> 122
   ugcccugugg acucaguucu gg 22
<210> 123
   <211> 22
   <212> RNA
   <213> Homo sapiens
<400> 123
   ugauauguuu gauauauuag gu 22
<210> 124
   <211> 23
   <212> RNA
   <213> Homo sapiens
<400> 124
   cccaguguuc agacuaccug uuc 23
<210> 125
   <211> 22
   <212> RNA
   <213> Homo sapiens
<400> 125
   ccuguucucc auuacuuggc uc 22
<210> 126
   <211> 20
   <212> RNA
   <213> Homo sapiens
<400> 126
   aaaagcuggg uugagagggu 20
<210> 127
   <211> 22
   <212> RNA
   <213> Homo sapiens
<400> 127
   aaagacauag uugcaagaug gg 22
<210> 128
   <211> 24
   <212> RNA
   <213> Homo sapiens
<400> 128
   aauccuugga accuaggugu gagu 24
<210> 129
   <211> 23
   <212> RNA
   <213> Homo sapiens
<400> 129
   ugagcaccac acaggccggg cgc 23
<210> 130
   <211> 22
   <212> RNA
   <213> Homo sapiens
<400> 130
   aauugcacuu uagcaauggu ga 22
<210> 131
   <211> 23
   <212> RNA
   <213> Homo sapiens
<400> 131
   ucaaggccag aggucccaca gca 23
<210> 132
   <211> 22
   <212> RNA
   <213> Homo sapiens
<400> 132
   cagcagcaau ucauguuuug aa 22
<210> 133
   <211> 23
   <212> RNA
   <213> Homo sapiens
<400> 133
   ucuuggagua ggucauuggg ugg 23
<210> 134
   <211> 20
   <212> RNA
   <213> Homo sapiens
<400> 134
   aaaagcuggg cugagaggcg 20
<210> 135
   <211> 23
   <212> RNA
   <213> Homo sapiens
<400> 135
   ucaaaaugua gaggaagacc cca 23
<210> 136
   <211> 22
   <212> RNA
   <213> Homo sapiens
<400> 136
   acccagguuc ccucuggccg ca 22
<210> 137
   <211> 23
   <212> RNA
   <213> Homo sapiens
<400> 137
   uggccggaug ggacaggagg cau 23
<210> 138
   <211> 21
   <212> RNA
   <213> Homo sapiens
<400> 138
   cagcagcaca cugugguuug u 21
<210> 139
   <211> 22
   <212> RNA
   <213> Homo sapiens
<400> 139
   aaugcaccug ggcaaggauu ca 22
<210> 140
   <211> 22
   <212> RNA
   <213> Homo sapiens
<400> 140
   aaagugcuuc ucuuuggugg gu 22
<210> 141
   <211> 22
   <212> RNA
   <213> Homo sapiens
<400> 141
   aagugcuucc uuuuagaggg uu 22
<210> 142
   <211> 22
   <212> RNA
   <213> Homo sapiens
<400> 142
   ugugucacuc gaugaccacu gu 22
<210> 143
   <211> 22
   <212> RNA
   <213> Homo sapiens
<400> 143
   gacuauagaa cuuucccccu ca 22
<210> 144
   <211> 22
   <212> RNA
   <213> Homo sapiens
<400> 144
   augcugacau auuuacuaga gg 22
<210> 145
   <211> 21
   <212> RNA
   <213> Homo sapiens
<400> 145
   uggguuuacg uugggagaac u 21
<210> 146
   <211> 22
   <212> RNA
   <213> Homo sapiens
<400> 146
   uuugugaccu gguccacuaa cc 22
<210> 147
   <211> 22
   <212> RNA
   <213> Homo sapiens
<400> 147
   auauaauaca accugcuaag ug 22
<210> 148
   <211> 22
   <212> RNA
   <213> Homo sapiens
<400> 148
   ugagguagga gguuguauag uu 22
<210> 149
   <211> 22
   <212> RNA
   <213> Homo sapiens
<400> 149
   ggagacgcgg cccuguugga gu 22
<210> 150
   <211> 22
   <212> RNA
   <213> Homo sapiens
<400> 150
   uaacacuguc ugguaaagau gg 22
<210> 151
   <211> 22
   <212> RNA
   <213> Homo sapiens
<400> 151
   ugcccugugg acucaguucu gg 22
<210> 152
   <211> 22
   <212> RNA
   <213> Homo sapiens
<400> 152
   aacauucaac cugucgguga gu 22
<210> 153
   <211> 22
   <212> RNA
   <213> Homo sapiens
<400> 153
   aaagaucugg aagugggaga ca 22
<210> 154
   <211> 24
   <212> RNA
   <213> Homo sapiens
<400> 154
   aauccuugga accuaggugu gagu 24
<210> 155
   <211> 21
   <212> RNA
   <213> Homo sapiens
<400> 155
   uaguagaccg uauagcguac g 21
<210> 156
   <211> 22
   <212> RNA
   <213> Homo sapiens
<400> 156
   acuggacuua gggucagaag gc 22
<210> 157
   <211> 17
   <212> RNA
   <213> Homo sapiens
<400> 157
   guggaccugg cugggac 17
<210> 158
   <211> 22
   <212> RNA
   <213> Homo sapiens
<400> 158
   gucauacacg gcucuccucu cu 22
<210> 159
   <211> 21
   <212> RNA
   <213> Homo sapiens
<400> 159
   cggggcagcu caguacagga u 21 <210> 160
   <211> 22
   <212> RNA
   <213> Homo sapiens
<400> 160
   aaucguacag ggucauccac uu 22
<210> 161
   <211> 22
   <212> RNA
   <213> Homo sapiens
<400> 161
   ugaaacauac acgggaaacc uc 22
<210> 162
   <211> 21
   <212> RNA
   <213> Homo sapiens
<400> 162
   gugucuuuug cucugcaguc a 21
<210> 163
   <211> 22
   <212> RNA
   <213> Homo sapiens
<400> 163
   aagugcuucc uuuuagaggg uu 22
<210> 164
   <211> 22
   <212> RNA
   <213> Homo sapiens
<400> 164
   caaaaaucuc aauuacuuuu gc 22
<210> 165
   <211> 22
   <212> RNA
   <213> Homo sapiens
<400> 165
   aaaaguaauu gcgguuuuug cc 22
<210> 166
   <211> 22
   <212> RNA
   <213> Homo sapiens
<400> 166
   gagcuuauuc auaaaagugc ag 22
<210> 167
   <211> 21
   <212> RNA
   <213> Homo sapiens
<400> 167
   uccgguucuc agggcuccac c 21
<210> 168
   <211> 21
   <212> RNA
   <213> Homo sapiens
<400> 168
   cgcgggugcu uacugacccu u 21
<210> 169
   <211> 21
   <212> RNA
   <213> Homo sapiens
<400> 169
   uuaauaucgg acaaccauug u 21
<210> 170
   <211> 22
   <212> RNA
   <213> Homo sapiens
<400> 170
   uauugcacuu gucccggccu gu 22
<210> 171
   <211> 21
   <212> RNA
   <213> Homo sapiens
<400> 171
   cugaccuaug aauugacagc c 21
<210> 172
   <211> 22
   <212> RNA
   <213> Homo sapiens
<400> 172
   uagcaccauc ugaaaucggu ua 22
<210> 173
   <211> 23
   <212> RNA
   <213> Homo sapiens
<400> 173
   uguaaacauc cuacacucuc agc 23
<210> 174
   <211> 22
   <212> RNA
   <213> Homo sapiens
<400> 174
   aagacgggag gaaagaaggg ag 22
<210> 175
   <211> 22
   <212> RNA
   <213> Homo sapiens
<400> 175
   auauaauaca accugcuaag ug 22

## Beschreibung

Die Erfindung betrifft die Verwendung bestimmter, im Blutserum-/Blutplasma zirkulierender microRNAs im Rahmen eines In-vitro-Verfahrens zur Unterscheidung zwischen Patienten, bei denen zur diagnostischen Identifikation einer Herzmuskelerkrankung eine Myokardbiopsie erforderlich ist, und Patienten, bei denen keine Myokardbiopsie erforderlich ist, sowie zur optionalen differentialdiagnostischen Unterscheidung einzelner nicht-ischämischer Kardiomyopathien oder Speichererkrankungen des Herzens gemäß dem Oberbegriff des Anspruchs 1.

Herz-Kreislauf-Erkrankungen sind heute in den westlichen Ländern die mit Abstand häufigste Todesursache. Die Inzidenz für Herzinsuffizienzen in Europa und den USA liegt bei 15 bzw. 12 Mio. Erkrankten. Mit ca. 5 Mio. (30%) erkrankten Patienten in Europa ist die dilatative Kardiomyopathie (DCM) die häufigste Ausprägung der nicht-ischämischen Kardiomyopathie. Die 5-Jahres-Überlebensrate bei dieser viral-entzündlich-induzierten Herzerkrankung liegt ohne spezifische Behandlung bei 50 %. Weiterhin entwickelte sich diese schwere Herzschädigung bei 45% aller transplantationspflichtigen Patienten auf der Grundlage einer vorliegenden DCM. Die hohe Inzidenz der nicht-ischämischen Kardiomyopathie und die enormen gesundheitsökonomischen Folgen dieser Erkrankungen erfordern eine frühzeitige und spezifische Diagnostik und eine darauf basierende zielführende Therapie. Dies gilt in gleicher Weise für Speichererkrankungen des Herzens, wie etwa durch Amyloidose bedingte Kardiomyopathien.

Unter einer Kardiomyopathie versteht man Erkrankungen der Herzmuskulatur selbst, die primär nicht als Folge von anderen Erkrankungen des kardiovaskulären Systems entstanden sind. Diese Erkrankungen beruhen also weder auf einer mechanischen Überlastung (z.B. durch zu hohen Blutdruck oder einem Klappenfehler), noch auf einer Mangeldurchblutung der Herzkranzgefäße (koronare Herzerkrankung).

Im Gegensatz zu den koronaren Herzerkrankungen mit vielfältigen Diagnosemöglichkeiten gibt es für die verschiedenen Formen der nicht-ischämischen Kardiomyopathien und Speichererkrankungen des Herzens bisher keine spezifischen, nicht-invasiven Diagnoseparameter. Aufgrund ihrer vielfältigen Entstehungsursachen können nicht-ischämische Kardiomyopathien und Speichererkrankungen des Herzens bisher nur mit invasiven Methoden (Myokardbiopsie) exakt diagnostiziert werden, mit dem Ziel, eine weitere Therapie-relevante Differenzierung der einzelnen Krankheitsbilder zu erreichen.

Derzeit geht man davon aus, dass neben den rein genetisch bedingten Formen Herzmuskelerkrankungen häufig durch eine Virusinfektion und/oder eine damit verbundene Entzündungsreaktion bedingt sind, wobei genetische Veranlagungen für den Verlauf der Erkrankung bedeutsam sein können. Diese sich überlappenden Krankheitsbilder sind bis heute pathogenetisch unzureichend aufgeklärt. Aus diesem Grund ist es wichtig, eine diagnostische Methodik zu entwickeln, die frühzeitig die unterschiedlichen Formen der klinisch nicht zu unterscheidenden Gruppen nicht-ischämischer Kardiomyopathien und Speichererkrankungen des Herzens diagnostizieren kann, um so frühestmöglich eine dem Patienten adäquate Therapie einleiten zu können.

Der Goldstandard für die Kardiomyopathie-Diagnostik ist die Herzmuskelbiopsie, welche aber nur in einzelnen kardiologischen Zentren und in wenigen Ländern und auch dort nur bei einer stark begrenzten Anzahl von Patienten durchgeführt wird. Zusätzlich erfolgt häufig nur eine histologische Begutachtung des Herzmaterials, ohne die notwendigen immunhistochemische Entzündungsdifferenzierung und die molekularbiologischen Untersuchungen auf virale Infektionen.

Bei der Häufigkeit der nicht-ischämischen Kardiomyopathien und Speichererkrankungen des Herzens und den enormen gesundheitsökonomischen Folgen wäre es aber wünschenswert, durch gering invasive Untersuchungsmethoden frühzeitig jene Patienten zu erkennen, bei denen der Verdacht auf eine viral-entzündliche Kardiomyopathie vorliegt. Um irreversible Myokardschäden vermeiden zu können sollten diese Patienten schnellstmöglich einer Myokardbiopsienentnahme unterzogen werden um sie bei Bedarf dann einer spezifischen Therapie zuführen zu können.

Weiterhin ist es wichtig zu wissen, welcher Patient auf welche Therapie anspricht. Erste Untersuchungen weisen darauf hin, dass das Vorliegen individueller Genexpressionsmuster möglicherweise mit einer genetischen Prädisposition assoziiert ist, welche die individuell erforderliche und wirksame Behandlung des Patienten beeinflussen kann.

Inzwischen sind microRNAs (miRNAs) als wichtige Regulatoren der genetischen Expression, auch in ihrer Bedeutung bei der Entstehung von Herzmuskelerkrankungen, erkannt worden. MicroRNAs sind einzelsträngige, kurze Ribonukleinsäure-Moleküle (RNA-Moleküle) mit 17 bis 24 Basen Länge, welche direkt in die Genregulation eingreifen. Hierbei werden insbesondere der Abbau der Ziel-RNA oder die Translation gesteuert. MicroRNAs bilden einen neuen Regelkreislauf krankheitsbedingter und gewebespezifischer Genexpression. In zunehmendem Maße werden zirkulierende microRNAs im menschlichen Serum als stabile Biomarker für die Diagnostik verschiedener Krankheiten und für das Monitoring angewandter Therapien verwendet.

Voellenkle et al. haben in der Publikation "MicroRNA signatures in peripheral blood mononuclear cells of chronic heart failure patients", Physiol. Genomics 42 (2010), Seiten 420-426, verschiedene microRNAs beschrieben, mittels derer gesunde Individuen von Patienten mit ischämischer Kardiomyopathie (ICM) oder mit nicht-ischämischer dilatativer Kardiomyopathie (NIDCM) unterschieden werden konnten. Eine Unterscheidung zwischen Patienten mit ischämischer Kardiomyopathie und Patienten mit nicht-ischämischer dilatativer Kardiomyopathie war auf der Basis der identifizierten microRNAs jedoch nicht möglich.

Ikeda et al. haben in der Publikation "Altered microRNA expression in human heart disease", Physiol. Genomics 31 (2007), Seiten 367-373, ebenfalls verschiedene microRNAs beschrieben, mittels derer zwischen unterschiedlichen Herzerkrankungen, nämlich der ischämischen Kardiomyopathie (ICM), der dilatativen Kardiomyopathie (DCM) und der Aortenstenose (AS; diese Erkrankung zählt als Klappenfehler nicht zu den Kardiomyopathien) differenziert werden konnte.

Prasad et al. haben in der Publikation "Unique microRNA profile in end-stage heart failure indicates alterations in specific cardiovascular signaling networks", J. Biol. Chem. 284 (2009), Seiten 27487-27499, acht microRNAs identifiziert, deren Konzentration im Gewebe im Falle eines Herzversagens verändert ist. Dabei wurden jedoch nur Patienten mit einer dilatativen Kardiomyopathie im Endstadium untersucht. Eine Unterscheidung verschiedener Herzmuskelerkrankungen untereinander war nicht Ziel dieser Publikation und wird entsprechend nicht beschrieben.

Kühl et al. haben in der Publikation "Differential Cardiac microRNA expression predicts the clinical course in human enterovirus cardiomyopathy", Circ. Heart Failure 8(3) (2015), Seiten 608-615, 16 microRNAs identifiziert, deren Konzentration im Gewebe prädiktiv für eine spontane Elimination oder die Langzeit-Persistenz einer myokardialen Enterovirusinfektion sind. Die Persistenz ist mit einer signifikant schlechteren Prognose korreliert und ist eine Indikation für eine Behandlung mit Interferon-beta. Diese Untersuchungen zeigen das diagnostisch-therapeutische Potential der microRNAs. Derartige Studien sind bisher nur für microRNAs aus Herzmuskelbiopsien gezeigt wurden.

Lassner et al. befassen sich in ihrer Publikation "Recent Advances in Molecular Diagnostics and Treatment of Heart Muscle Diseases", JASMI, 3(2) (2013), Seiten 98-109 mit der Diagnostik und Behandlung von Kardiomyopathien unter Verwendung von microRNAs.

Aus der WO 2013/127782 A2 ist die Verwendung spezifischer Nukleinsäuren als Marker zur Identifizierung einzelner Formen nicht-ischämischer Kardiomyopathien oder Speichererkrankungen des Herzens bekannt. Dabei wurden in jener internationalen Patentanmeldung vorrangig Herzmuskelbiopsien und periphere Blutzellen untersucht.

Häufig treten auch Infektionen des Herzmuskels auf, die zunächst noch nicht zu einer Kardiomyopathie führen. Im klinischen Befund zeigt sich also ein normal großes und normal leistungsfähiges Herz, das beispielsweise durch ein Virus infiziert ist. Diese Infektion kann nachfolgend zu einer Kardiomyopathie führen; sie stellt folglich eine Vorform der Kardiomyopathie dar. Frühzeitig erkannt und behandelt, lässt sich der Ausbruch einer Kardiomyopathie jedoch vermeiden.

Bislang ist es gelungen, microRNA-basierte Diagnosen durch die Genprofile im Herzgewebe zu erstellen, mittels derer unterschiedliche Typen nicht-ischämischer Kardiomyopathien, Speichererkrankungen des Herzens und Infektionen des Herzmuskels ohne kardiomyopathische Ausprägungen identifiziert und voneinander unterschieden werden können (vgl. beispielsweise WO 2013/127782 A2).

Der vorliegenden Erfindung liegt die Aufgabe zugrunde, geeignete microRNAs für eine entsprechende Identifizierung von Patienten mit Verdacht auf eine nicht-ischämische Kardiomyopathie, Speichererkrankungen des Herzens oder Infektionen des Herzmuskels ohne kardiomyopathische Ausprägungen anzugeben.

Diese Aufgabe wird mit einem In-vitro-Verfahren zur Unterscheidung zwischen Patienten, bei denen zur diagnostischen Identifikation einer Herzmuskelerkrankung eine Myokardbiopsie erforderlich ist, und Patienten, bei denen keine Myokardbiopsie erforderlich ist, sowie zur optionalen differentialdiagnostischen Unterscheidung einzelner nicht-ischämischer Kardiomyopathien oder Speichererkrankungen des Herzens gelöst.

Das Verfahren dient insbesondere dazu, Ausgangsdaten für eine nachfolgende Diagnose bzw. diagnostische Klassifikation bereitzustellen.

Im Rahmen dieses Verfahrens wird eine (optional zunächst aufbereitete) Blutserum- oder Blutplasmaprobe (nachfolgend verkürzt als Blutserum-/Blutplasmaprobe bezeichnet) eines Patienten unter Verwendung eines diagnostischen Profils analysiert wird. Das diagnostische Profil umfasst mindestens 2 microRNAs oder synthetische Nukleinsäuren mit identischen oder komplementären Sequenzen umfasst, die aus der Gruppe bestehend aus den folgenden microRNAs ausgewählt sind: hsa-let-7f-5p (SEQ ID NO: 10), hsa-miR-103a-3p (SEQ ID NO: 11), hsa-miR-197-3p (SEQ ID NO: 12), hsa-miR-215-5p (SEQ ID NO: 13), hsa-miR-223-3p (SEQ ID NO: 14), hsa-miR-23a-3p (SEQ ID NO: 15), hsa-miR-330-3p (SEQ ID NO: 16), hsa-miR-337-5p (SEQ ID NO: 17), hsa-miR-339-3p (SEQ ID NO: 18), hsa-miR-339-5p (SEQ ID NO: 19), hsa-miR-411-5p (SEQ ID NO: 20), hsa-miR-454-3p (SEQ ID NO: 21), hsa-miR-485-3p (SEQ ID NO: 22), hsa-miR-487b-3p (SEQ ID NO: 23), hsa-miR-494-3p (SEQ ID NO: 24), hsa-miR-505-3p (SEQ ID NO: 25), hsa-miR-628-5p (SEQ ID NO: 26), hsa-miR-671-3p (SEQ ID NO: 27), hsa-miR-744-5p (SEQ ID NO: 28), hsa-miR-889-3p (SEQ ID NO: 29), mmu-miR-495-3p (SEQ ID NO: 30).

Demzufolge werden mehrere (mindestens 2) definierte microRNAs als Marker zur Identifizierung biopsiepflichtiger Patienten verwendet.

Als "identisch" wird eine Sequenz dann angesehen, wenn sie zu mindestens 90 %, insbesondere mindestens 95 %, insbesondere mindestens 97 %, insbesondere mindestens 98 %, insbesondere mindestens 99 %, insbesondere mindestens 99,5 % und ganz besonders zu mindestens 99,9 % mit der jeweils betrachteten Sequenz übereinstimmt.

Die vorgenannten microRNA-Bezeichnungen sind dem Fachmann allgemein bekannte eindeutige Bezeichnungen unterschiedlicher microRNA-Sequenzen. Die jeweils hinter diesen Bezeichnungen stehenden Sequenzen können beispielsweise in der frei zugänglichen Datenbank miRBase (unter der Internet-Adresse http://www.mirbase.org erreichbar) abgefragt werden; sie sind zudem im der beigefügten Sequenzliste angegeben.

Sofern einzelne der vorgenannten Bezeichnungen sowohl für eine Stamm-Schlaufen-Struktur als auch für eine reife microRNA-Sequenz verwendet werden, ist jeweils die reife microRNA-Sequenz, die dem Fachmann auch unter der englischsprachigen Bezeichnung "mature sequence" bekannt ist, als die hinter der jeweiligen Abkürzung stehende Sequenz zu verstehen.

Die beanspruchte Erfindung beruht auf dem Konzept, ein diagnostisches Profil aus microRNAs als Marker zu verwenden, um das zunächst eine Unterscheidung zwischen gesunden (nicht biopsiepflichtigen) und an einer Kardiomyopathie erkrankten Patienten zu unterscheiden sowie optional das Vorhandensein eines Subtyps einer nicht-ischämischen Kardiomyopathie oder einer Speichererkrankungen des Herzens zu identifizieren. Es geht vorliegend primär darum, gesunde Patienten von kranken Patienten zu unterscheiden. In einem zweiten Schritt können die biopsiepflichtigen Patienten in die spezifischen Untergruppen nicht-ischämischer Kardiomyopathien und Untergruppen von Speichererkrankungen des Herzens klassifiziert werden.

Wie erläutert, werden die microRNAs der obigen Liste und aller nachfolgenden Listen in Form diagnostisch relevanter Profile eingesetzt. Ein solches Profil kann beispielsweise aus mindestens oder genau 2, 3, 4, 5, 6, 7, 8, 9, 10, 11, 12, 13, 14, 15, 20, 30, 40, 50 oder aber allen microRNAs der obigen Liste bzw. den jeweiligen nachfolgenden Listen bestehen.

In einer Variante umfasst das diagnostische Profil zusätzlich mindestens 3 microRNAs oder synthetische Nukleinsäuren mit identischen oder komplementären Sequenzen, die aus der Gruppe bestehend aus den folgenden microRNAs ausgewählt sind: hsa-miR-1274B (SEQ ID NO: 31), hsa-miR-136-3p (SEQ ID NO: 32), hsa-miR-187-3p (SEQ ID NO: 33), hsa-miR-199a-5p (SEQ ID NO: 34), hsa-miR-215-5p (SEQ ID NO: 35), hsa-mir-422a (SEQ ID NO: 36), hsa-miR-4449 (SEQ ID NO: 37), hsa-miR-4674 (SEQ ID NO: 38), hsa-miR-5096 (SEQ ID NO: 39), hsa-miR-548c-3p (SEQ ID NO: 40), hsa-miR-548c-5p (SEQ ID NO: 41), hsa-miR-650 (SEQ ID NO: 42). Dann eignet sich das diagnostische Profil auch zur Differentialdiagnose bzw. zur Identifikation einer Adenovirus-induzierten Kardiomyopathie.

In einer Variante umfasst das diagnostische Profil zusätzlich mindestens 3 microRNAs oder synthetische Nukleinsäuren mit identischen oder komplementären Sequenzen, die aus der Gruppe bestehend aus den folgenden microRNAs ausgewählt sind: hsa-miR-1180-3p (SEQ ID NO: 43), hsa-miR-1-3p (SEQ ID NO: 44), hsa-miR-140-5p (SEQ ID NO: 45), hsa-miR-150-3p (SEQ ID NO: 46), hsa-miR-16-5p (SEQ ID NO: 47), hsa-miR-215-5p (SEQ ID NO: 48), hsa-miR-25-3p (SEQ ID NO: 49), hsa-miR-30a-5p (SEQ ID NO: 50), hsa-miR-30e-5p (SEQ ID NO: 51), hsa-miR-3138 (SEQ ID NO: 52), hsa-miR-3622b-5p (SEQ ID NO: 53), hsa-miR-3922-5p (SEQ ID NO: 54), hsa-mir-422a (SEQ ID NO: 55), hsa-miR-4274 (SEQ ID NO: 56), hsa-miR-4303 (SEQ ID NO: 57), hsa-miR-4419b (SEQ ID NO: 58), hsa-miR-4429 (SEQ ID NO: 59), hsa-miR-4467 (SEQ ID NO: 60), hsa-miR-4651 (SEQ ID NO: 61), hsa-miR-4716-5p (SEQ ID NO: 62), hsa-miR-548b-5p (SEQ ID NO: 63), hsa-miR-548c-3p (SEQ ID NO: 64), hsa-miR-597-5p (SEQ ID NO: 65), hsa-miR-601 (SEQ ID NO: 66), hsa-miR-629-3p (SEQ ID NO: 67), hsa-miR-660-5p (SEQ ID NO: 68), hsa-miR-886-3p (SEQ ID NO: 69), hsa-miR-923 (SEQ ID NO: 70), mmu-miR-374-5p (SEQ ID NO: 71). Dann eignet sich das diagnostische Profil auch zur Differentialdiagnose bzw. zur Identifikation einer Enterovirus-(Coxsackievirus-)induzierten Kardiomyopathie.

In einer Variante umfasst das diagnostische Profil zur Identifikation einer Amyloidose des Herzens zusätzlich mindestens 3 microRNAs oder synthetische Nukleinsäuren mit identischen oder komplementären Sequenzen, die aus der Gruppe bestehend aus den folgenden microRNAs ausgewählt sind: hsa-miR-103a-3p (SEQ ID NO: 72), hsa-miR-106b-5p (SEQ ID NO: 73), hsa-miR-126-3p (SEQ ID NO: 74), hsa-miR-126-5p (SEQ ID NO: 75), hsa-miR-1274A (SEQ ID NO: 76), hsa-miR-1274B (SEQ ID NO: 77), hsa-miR-132-3p (SEQ ID NO: 78), hsa-miR-140-3p (SEQ ID NO: 79), hsa-miR-142-3p (SEQ ID NO: 80), hsa-miR-146b-5p (SEQ ID NO: 81), hsa-miR-150-5p (SEQ ID NO: 82), hsa-miR-15b-5p (SEQ ID NO: 83), hsa-miR-181b-5p (SEQ ID NO: 84), hsa-miR-186-5p (SEQ ID NO: 85), hsa-miR-21-5p (SEQ ID NO: 86), hsa-miR-222-3p (SEQ ID NO: 87), hsa-miR-223-5p (SEQ ID NO: 88), hsa-miR-24-3p (SEQ ID NO: 89), hsa-miR-26b-5p (SEQ ID NO: 90), hsa-miR-30a-3p (SEQ ID NO: 91), hsa-miR-30d-5p (SEQ ID NO: 92), hsa-miR-320a (SEQ ID NO: 93), hsa-miR-320c (SEQ ID NO: 94), hsa-miR-328-3p (SEQ ID NO: 95), hsa-miR-375 (SEQ ID NO: 96), hsa-miR-378-5p (SEQ ID NO: 97), hsa-mir-423 (SEQ ID NO: 98), hsa-miR-4286 (SEQ ID NO: 99), hsa-miR-451a (SEQ ID NO: 100), hsa-miR-4535 (SEQ ID NO: 101), hsa-miR-483-5p (SEQ ID NO: 102), hsa-miR-518d-3p (SEQ ID NO: 103), hsa-miR-532-5p (SEQ ID NO: 104), hsa-miR-590-5p (SEQ ID NO: 105), hsa-miR-601 (SEQ ID NO: 106), hsa-miR-660-5p (SEQ ID NO: 107), hsa-miR-885-5p (SEQ ID NO: 108), hsa-miR-92a-3p (SEQ ID NO: 109), hsa-miR-99b-5p (SEQ ID NO: 110). Dann eignet sich das diagnostische Profil auch zur Differentialdiagnose bzw. zur Identifikation einer Amyloidose des Herzens.

In einer weiteren Variante lässt sich eine solche Amyloidose noch feiner unterteilen und entsprechend genauer diagnostizieren. So lässt sich eine Subklassifizierung für die Speichererkrankung Amyloidose durchführen, in der eine Unterscheidung zwischen ATTR-Amyloidose und Lambda-Amyloidose getroffen werden kann.

So sind die folgenden microRNAs zur Identifikation einer Lambda-Amyloidose des Herzens geeignet: hsa-miR-106b-5p (SEQ ID NO: 73), hsa-miR-1274A (SEQ ID NO: 76), hsa-miR-30a-3p (SEQ ID NO: 91), hsa-miR-4535 (SEQ ID NO: 101). In einer Variante umfasst das diagnostische Profil daher zusätzlich mindestens 3 microRNAs oder synthetische Nukleinsäuren mit identischen oder komplementären Sequenzen, die aus der Gruppe bestehend aus den vorstehend genannten microRNAs ausgewählt sind.

Die folgenden microRNAs sind zu Identifikation einer ATTR-Amyloidose des Herzens geeignet: hsa-miR-140-3p (SEQ ID NO: 79), hsa-miR-181b-5p (SEQ ID NO: 84), hsa-miR-222-3p (SEQ ID NO: 87), hsa-miR-223-5p (SEQ ID NO: 88). In einer Variante umfasst das diagnostische Profil daher zusätzlich mindestens 3 microRNAs oder synthetische Nukleinsäuren mit identischen oder komplementären Sequenzen, die aus der Gruppe bestehend aus den vorstehend genannten microRNAs ausgewählt sind.

In einer Variante umfasst das diagnostische Profil zusätzlich mindestens 3 microRNAs oder synthetische Nukleinsäuren mit identischen oder komplementären Sequenzen, die aus der Gruppe bestehend aus den folgenden microRNAs ausgewählt sind: hsa-miR-139-3p (SEQ ID NO: 111), hsa-miR-210-3p (SEQ ID NO: 112), hsa-miR-296-5p (SEQ ID NO: 113), hsa-miR-486-3p (SEQ ID NO: 114), hsa-miR-500a-5p (SEQ ID NO: 115), hsa-miR-542-3p (SEQ ID NO: 116). Dann eignet sich das diagnostische Profil auch zur Differentialdiagnose bzw. zur Identifikation einer kardialen Sarkoidose/Riesenzellenmyokarditis des Herzens.

In einer Variante umfasst das diagnostische Profil zusätzlich mindestens 3 microRNAs oder synthetische Nukleinsäuren mit identischen oder komplementären Sequenzen, die aus der Gruppe bestehend aus den folgenden microRNAs ausgewählt sind: hsa-miR-103b (SEQ ID NO: 117), hsa-miR-125a-5p (SEQ ID NO: 118), hsa-miR-1271-5p(SEQ ID NO: 119), hsa-miR-1274B (SEQ ID NO: 120), hsa-miR-138-1-3p (SEQ ID NO: 121), hsa-miR-146b-3p (SEQ ID NO: 122), hsa-miR-190a-5p (SEQ ID NO: 123), hsa-miR-199a-5p (SEQ ID NO: 124), hsa-miR-26b-3p (SEQ ID NO: 125), hsa-miR-320c (SEQ ID NO: 126), hsa-miR-3617-5p (SEQ ID NO: 127), hsa-miR-362-5p (SEQ ID NO: 128), hsa-miR-3663-3p (SEQ ID NO: 129), hsa-miR-367-3p (SEQ ID NO: 130), hsa-miR-3922-5p (SEQ ID NO: 131), hsa-miR-424-5p (SEQ ID NO: 132), hsa-miR-432-5p (SEQ ID NO: 133), hsa-miR-4429 (SEQ ID NO: 134), hsa-miR-4698 (SEQ ID NO: 135), hsa-miR-4726-3p (SEQ ID NO: 136), hsa-miR-4743-5p (SEQ ID NO: 137), hsa-miR-497-5p (SEQ ID NO: 138), hsa-miR-502-3p (SEQ ID NO: 139), hsa-miR-520d-3p (SEQ ID NO: 140), hsa-miR-520f-3p (SEQ ID NO: 141), hsa-miR-597-5p (SEQ ID NO: 142), hsa-miR-625-3p (SEQ ID NO: 143), hsa-miR-628-5p (SEQ ID NO: 144), hsa-miR-629-5p (SEQ ID NO: 145), hsa-miR-758-3p (SEQ ID NO: 146), mmu-miR-374-5p (SEQ ID NO: 147). Dann eignet sich das diagnostische Profil auch zur Differentialdiagnose bzw. zur Identifikation einer Erythrovirus-induzierten Kardiomyopathie.

In einer Variante umfasst das diagnostische Profil zusätzlich mindestens 3 microRNAs oder synthetische Nukleinsäuren mit identischen oder komplementären Sequenzen, die aus der Gruppe bestehend aus den folgenden microRNAs ausgewählt sind: hsa-let-7e-5p (SEQ ID NO: 148), hsa-miR-139-3p (SEQ ID NO: 149), hsa-miR-141-3p (SEQ ID NO: 150), hsa-miR-146b-3p (SEQ ID NO: 151), hsa-miR-181c-5p (SEQ ID NO: 152), hsa-miR-3156-5p (SEQ ID NO: 153), hsa-miR-362-5p (SEQ ID NO: 154), hsa-miR-411-5p (SEQ ID NO: 155), hsa-mir-422a (SEQ ID NO: 156), hsa-miR-4535 (SEQ ID NO: 157), hsa-miR-485-3p (SEQ ID NO: 158), hsa-miR-486-3p (SEQ ID NO: 159), hsa-miR-487b-3p (SEQ ID NO: 160), hsa-miR-494-3p (SEQ ID NO: 161), hsa-miR-511-5p (SEQ ID NO: 162), hsa-miR-520f-3p (SEQ ID NO: 163), hsa-miR-548c-3p (SEQ ID NO: 164), hsa-miR-548c-5p (SEQ ID NO: 165), hsa-miR-590-5p (SEQ ID NO: 166), hsa-miR-671-3p (SEQ ID NO: 167), hsa-miR-886-3p (SEQ ID NO: 168), hsa-miR-889-3p (SEQ ID NO: 169), hsa-miR-92a-3p (SEQ ID NO: 170). Dann eignet sich das diagnostische Profil auch zur Differentialdiagnose bzw. zur Identifikation einer ciHHV6-induzierten Kardiomyopathie.

In einer Variante umfasst das diagnostische Profil zusätzlich mindestens 3 microRNAs oder synthetische Nukleinsäuren mit identischen oder komplementären Sequenzen, die aus der Gruppe bestehend aus den folgenden microRNAs ausgewählt sind: hsa-miR-192-5p (SEQ ID NO: 171), hsa-miR-29a-3p (SEQ ID NO: 172), hsa-miR-30c-5p (SEQ ID NO: 173), hsa-miR-483-5p (SEQ ID NO: 174), mmu-miR-374-5p (SEQ ID NO: 175). Dann eignet sich das diagnostische Profil auch zur Differentialdiagnose bzw. zur Identifikation einer HHV6-induzierten Kardiomyopathie.

Es ist folglich möglich, je nach zu diagnostizierender Erkrankung, unterschiedliche microRNAs in diagnostischen Profilen verschiedener microRNAs einzusetzen. Es ist aber auch möglich, ein komplexeres diagnostisches Profil unterschiedlicher microRNAs für die Diagnose der verschiedenen zu diagnostizierenden Erkrankungen einzusetzen. Dies hat den Vorteil, dass mit einem einzigen diagnostischen Profil unterschiedlichste Diagnosen erstellt werden können, ein derartiges Profil also universell innerhalb der gegebenen Fragestellung einsetzbar ist.

Es wurden verschiedene Gruppierungen an nicht-ischämischen Kardiomyopathien oder Speichererkrankungen des Herzens gebildet, deren Unterscheidung voneinander mit den vorstehend aufgelisteten microRNAs möglich ist: Adenovirus-induzierte Kardiomyopathie, Enterovirus-(Coxsackievirus-)induzierte Kardiomyopathie, HHV6-Virus-induzierte Kardiomyopathie, chromosomal-integriertes HHV6 (ciHHV6)-induzierte Kardiomyopathie, Erythrovirus-induzierte Kardiomyopathie, Myokarditis mit kardialen Riesenzellen und kardiale Amyloidose. Wie erläutert, kann optional eine Unterscheidung in Lambda- und ATTR-Amyloidose erfolgen.

Vorzugsweise werden nur Erkrankungen des Menschen berücksichtigt. So sind auch die microRNAs der obigen Liste grundsätzlich als humane Sequenzen zu verstehen.

In einer Variante sind die als Marker im Rahmen eines diagnostischen Profils einzusetzenden microRNAs ausgewählt aus der Gruppe bestehend aus all denjenigen microRNAs, denen für die zur Unterscheidung zwischen Patienten, bei denen zur diagnostischen Identifikation einer Herzmuskelerkrankung eine Myokardbiopsie erforderlich ist, und Patienten, bei denen keine Myokardbiopsie erforderlich ist, oder die zur Diagnose einer spezifischen nicht-ischämischen Kardiomyopathie oder einer spezifischen Speichererkrankung des Herzens in den Untersuchungen, die in den unten dargestellten Beispielen erläutert werden, der Punktwert 1 und/oder 2 und/oder der Signifikanzwert 1 und/oder 2 zugeordnet wurde.

In einer Variante sind die als Marker einzusetzenden Nukleinsäuren ausgewählt aus der Gruppe, die gerade nicht diejenigen microRNAs umfasst, denen für die Unterscheidung zwischen Patienten, bei denen zur diagnostischen Identifikation einer Herzmuskelerkrankung eine Myokardbiopsie erforderlich ist, und Patienten, bei denen keine Myokardbiopsie erforderlich ist, oder für die Diagnose einer spezifischen nicht-ischämischen Kardiomyopathie oder einer Speichererkrankung des Herzens in den Untersuchungen, die in den unten dargestellten Beispielen erläutert werden, der Punktwert 3 oder 4 und/oder der Signifikanzwert 4 oder 5 zugeordnet wurde. Dabei können sowohl einzelne, mehrere oder alle dieser mit den Punktwerten 3 oder 4 bzw. Signifikanz werden 4 oder 5 versehenen microRNAs vom Schutzbereich ausgeschlossen sein.

In einer Variante wird das In-vitro-Verfahren derart durchgeführt, dass die nachfolgend erläuterten Schritte ausgeführt werden. Zunächst wird eine gegebenenfalls aufbereitete Probe bereitgestellt, die von einem Patienten, bei dem ein Verdacht auf das Vorliegen einer nicht-ischämischen Kardiomyopathie oder einer Speichererkrankung des Herzens vorliegt, gewonnen wurde. Anschließend wird die Probe direkt oder nach vorangegangener biochemischer Modifikation mit mindestens zwei Sonden in Kontakt gebracht, wobei die Sonden jeweils eine Sequenz aufweisen, welche den Sequenzen der microRNAs aus den obigen Listen entspricht oder zu diesen komplementär ist. Dieser Kontakt erfolgt unter Bedingungen, die eine Hybridisierung zwischen in der Probe enthaltenen microRNAs auf der einen Seite und den Sonden auf der anderen Seite erlauben. Danach wird ermittelt, ob eine Hybridisierung zwischen den microRNAs der Probe und den Sonden erfolgt ist. Anschließend wird anhand dieses Hybridisierungsergebnisses bestimmt, welche microRNA(s) in der Probe vorhanden ist/sind.

Die Probe kann eine Blutserum-/Blutplasmaprobe eines Patienten sein. Alternativ kann es sich bei der Probe um einen Total-RNA-Extrakt handeln, der aus einer Blutserum-/Blutplasmaprobe des Patienten stammt, die wiederum anteilig aus microRNAs besteht. Hierbei ist es unerheblich, ob die RNA direkt aus der gesamten Blutserum-/Blutplasmaprobe isoliert wird, oder vorab eine spezifische Extraktion der Exosomen erfolgt, die nur einen definierten Anteil der zirkulierenden microRNAs enthalten. Aus dieser Probe kann dann vor Beginn des Verfahrens ein RNA-Extrakt gewonnen, das als Probe im vorliegend beanspruchten Verfahren verwendet wird. Es kann auch ein Direktnachweis der zirkulierenden microRNA aus den Blutserum-/Blutplasmaproben erfolgen.

Vorzugsweise geschieht die Ermittlung einer erfolgten Hybridisierung in semi-quantitativer oder in quantitativer Weise.

Werden die vorgenannten microRNAs im Rahmen einer microRNA-Profil-Diagnostik eingesetzt, ist es möglich, Patienten mit einer nicht-ischämischen Kardiomyopathie, einer Speichererkrankung des Herzens oder einer Infektion des Herzmuskels auch ohne eine Myokardbiopsie frühzeitig zu identifizieren und anschließend in eine bestimmte Subgruppe zu klassifizieren. Nach der Myokardbiopsie-Diagnostik kann dann dem jeweiligen Krankheitsbild entsprechend gezielt behandelt werden.

Dazu werden vorzugsweise die Expressionsraten der vorgenannten microRNAs in quantitativer oder semi-quantitativer Weise bestimmt. Aus einer erhöhten Expression bestimmter microRNAs und/oder einer verminderten Expression anderer bestimmter microRNAs lässt sich dann eine bestimmte nicht-ischämische Kardiomyopathie oder eine Speichererkrankung des Herzens diagnostizieren.

Die vorgenannten Nukleinsäuren (microRNAs) haben sich dabei als besonders gut geeignet für die spezifische Diagnostik einzelner nicht-ischämischer Kardiomyopathien und Speichererkrankungen des Herzens herausgestellt. Einige der Nukleinsäuren sind für bestimmte zu diagnostizierende Erkrankungen derart spezifisch, dass sie bereits allein als spezifischer Marker eingesetzt werden können. Andere Nukleinsäuren sind nicht für eine einzelne zu diagnostizierende Erkrankung spezifisch, sondern beispielsweise für zwei zu diagnostizierende Erkrankungen. Daher werden solche Nukleinsäuren vorzugsweise nicht alleine, sondern in Kombination mit anderen Nukleinsäuren aus der obigen Liste eingesetzt. Aber auch bei spezifischen Nukleinsäuren bietet es sich zur Verfeinerung der Diagnostik an, diese nicht einzeln, sondern in Kombination mit anderen Nukleinsäuren der obigen Liste einzusetzen. Auf diese Weise lässt sich die Aussagekraft diagnostischer Tests stabilisieren.

Statt den genannten microRNAs können auch synthetische Nukleinsäuremoleküle eingesetzt werden, die eine identische oder komplementäre Sequenz zu den vorgenannten microRNAs aufweisen.

Die Erfindung betrifft auch die Verwendung der vorstehend aufgelisteten microRNAs im Rahmen diagnostischer Profile zur Analyse einer Blutserum-/Blutplasmaprobe eines Patienten, um zwischen Patienten, bei denen zur diagnostischen Identifikation einer Herzmuskelerkrankung eine Myokardbiopsie erforderlich ist, und Patienten, bei denen keine Myokardbiopsie erforderlich ist, unterscheiden zu können sowie um optional eine differentialdiagnostische Unterscheidung einzelner nicht-ischämischer Kardiomyopathien oder Speichererkrankungen des Herzens vornehmen zu können.

Des Weiteren wird ein diagnostisches System zur Unterscheidung zwischen Patienten offenbart, bei denen zur diagnostischen Identifikation einer Herzmuskelerkrankung eine Myokardbiopsie erforderlich ist, und Patienten, bei denen keine Myokardbiopsie erforderlich ist, sowie zur optionalen differentialdiagnostischen Unterscheidung einzelner nicht-ischämischer Kardiomyopathien oder Speichererkrankungen des Herzens. Ein solches diagnostisches System weist mindestens zwei Sonden aufweist, die jeweils eine Sequenz aufweisen, welche einer Sequenz einer microRNA aus der Gruppe bestehend aus den folgenden microRNAs entspricht oder zu dieser komplementär ist: hsa-miR-487b-3p (SEQ ID NO: 23), hsa-miR-494-3p (SEQ ID NO: 24), hsa-miR-889-3p (SEQ ID NO: 29), mmu-miR-495-3p (SEQ ID NO: 30). Hinsichtlich des Begriffs "entsprechen" wird auf die obigen Erläuterungen in Bezug auf den Begriff "identisch" verwiesen, die in analoger Weise anzuwenden sind.

In einer Variante umfasst das diagnostische System zusätzlich mindestens drei Sonden, die jeweils eine Sequenz aufweisen, welche einer Sequenz einer microRNA aus der Gruppe bestehend aus den folgenden microRNAs entspricht oder zu dieser komplementär ist: hsa-miR-1274B (SEQ ID NO: 31), hsa-miR-4449 (SEQ ID NO: 37), hsa-miR-4674 (SEQ ID NO: 38), hsa-miR-5096 (SEQ ID NO: 39), hsa-miR-548c-5p (SEQ ID NO: 41), hsa-miR-1180-3p (SEQ ID NO: 43), hsa-miR-1-3p (SEQ ID NO: 44), hsa-miR-16-5p (SEQ ID NO: 47), hsa-miR-3138 (SEQ ID NO: 52), hsa-miR-3622b-5p (SEQ ID NO: 53), hsa-miR-3922-5p (SEQ ID NO: 54), hsa-miR-4274 (SEQ ID NO: 56), hsa-miR-4303 (SEQ ID NO: 57), hsa-miR-4419b (SEQ ID NO: 58), hsa-miR-4429 (SEQ ID NO: 59), hsa-miR-4467 (SEQ ID NO: 60), hsa-miR-4651 (SEQ ID NO: 61), hsa-miR-4716-5p (SEQ ID NO: 62), hsa-miR-548b-5p (SEQ ID NO: 63), hsa-miR-597-5p (SEQ ID NO: 65), hsa-miR-923 (SEQ ID NO: 70), mmu-miR-374-5p (SEQ ID NO: 71), miR-1274A (SEQ ID NO: 76), hsa-miR-1274B (SEQ ID NO: 77), hsa-miR-223-5p (SEQ ID NO: 88), hsa-miR-328-3p (SEQ ID NO: 95), hsa-miR-378-5p (SEQ ID NO: 97), hsa-mir-423 (SEQ ID NO: 98), hsa-miR-4286 (SEQ ID NO: 99), hsa-miR-4535 (SEQ ID NO: 101), hsa-miR-210-3p (SEQ ID NO: 112), hsa-miR-103b (SEQ ID NO: 117), hsa-miR-1274B (SEQ ID NO: 120), hsa-miR-138-1-3p (SEQ ID NO: 121), hsa-miR-146b-3p (SEQ ID NO: 122), hsa-miR-190a-5p (SEQ ID NO: 123), hsa-miR-26b-3p (SEQ ID NO: 125), hsa-miR-3617-5p (SEQ ID NO: 127), hsa-miR-3663-3p (SEQ ID NO: 129), hsa-miR-3922-5p (SEQ ID NO: 131), hsa-miR-424-5p (SEQ ID NO: 132), hsa-miR-4429 (SEQ ID NO: 134), hsa-miR-4698 (SEQ ID NO: 135), hsa-miR-4726-3p (SEQ ID NO: 136), hsa-miR-4743-5p (SEQ ID NO: 137), hsa-miR-597-5p (SEQ ID NO: 142), hsa-miR-629-5p (SEQ ID NO: 145), hsa-miR-758-3p (SEQ ID NO: 146), mmu-miR-374-5p (SEQ ID NO: 147), hsa-miR-146b-3p (SEQ ID NO: 151), hsa-miR-3156-5p (SEQ ID NO: 153), hsa-miR-4535 (SEQ ID NO: 157), hsa-miR-487b-3p (SEQ ID NO: 160), hsa-miR-494-3p (SEQ ID NO: 161), hsa-miR-511-5p (SEQ ID NO: 162), hsa-miR-548c-5p (SEQ ID NO: 165), hsa-miR-889-3p (SEQ ID NO: 169), mmu-miR-374-5p (SEQ ID NO: 175).

Vorzugsweise weist das diagnostische System mindestens oder genau 2, 3, 4, 5, 6, 7, 8, 9, 10, 11, 12, 13, 14, 15, 20, 25, 30, 35, 40, 45, 50 der vorstehend aufgelisteten microRNAs als Sonden auf, die jeweils in mehreren Kopien in dem diagnostischen System enthalten sein können. Ferner ist es möglich, dass sämtliche microRNAs der obigen Liste in identischer oder komplementärer Sequenz im diagnostischen System als Sonde enthalten sind. Das diagnostische System weist also eine bestimmbare Anzahl diagnostisch relevanter microRNA-Sequenzen auf, die im System ein diagnostisch relevantes microRNA-Profil darstellen.

Vorzugsweise liegt das diagnostische System in Form eines Kits zur Durchführung einer Polymerase-Kettenreaktion (PCR) vor, wobei die Sonden als Primer in Lösung bereitgestellt werden. Auf diese Weise ist es besonders einfach möglich, in quantitativer Weise zu arbeiten, da mit Hilfe des Kits eine quantitative PCR durchgeführt werden kann. Durch den Einsatz von Trägerplatten mit einer großen Anzahl an Vertiefungen (beispielsweise 384 Vertiefungen) können mit einem derartigen System zahlreiche PCRs gleichzeitig durchgeführt werden, so dass sich dieses System auch für eine große Anzahl an Sonden eignet.

Die Bestimmung der Expressionslevels erfolgt in einer Variante durch die Messung der einzelnen Ziel-microRNAs im Verhältnis zu einer oder mehrerer konstitutiv exprimierter microRNAs, sogenannte Housekeeping-microRNAs, in der jeweiligen Blutserum-/Blutplasmaprobe.

In einer alternativen bevorzugten Ausgestaltung liegt das diagnostische System in Form eines Nukleinsäure-Chips vor. Dabei kann insbesondere vorgesehen sein, dass mindestens 5 Sonden für jede nachzuweisende Nukleinsäure auf den Chip aufgetragen sind. Auf diese Weise lässt sich die Expression von microRNAs bevorzugt semi-quantitativ messen. Ein Chip weist den Vorteil auf, dass das Vorhandensein zahlreicher verschiedener microRNAs in der untersuchten Probe gleichzeitig detektiert werden kann. Dabei ist die Anzahl gleichzeitig detektierbarer microRNAs noch weitaus größer als im Falle einer PCR. Insbesondere bei einer größeren Anzahl an Sonden (beispielsweise mehr als 25 Sonden), bietet sich die Verwendung eines Chips aus Effizienzgründen besonders an.

Zudem wird ein Medikament zur Behandlung von nicht-ischämischen Kardiomyopathien oder Speichererkrankungen des Herzens offenbart, das als pharmazeutisch aktive Substanz mindestens eine Nukleinsäure enthält, die eine Sequenz aufweist, die identisch oder komplementär zu der Sequenz einer der microRNAs ist, die im Zusammenhang mit dem diagnostischen System aufgelistet sind.

Ein solches Medikament kann dazu dienen, den Gehalt einer bestimmten microRNA im Blut bzw. in den Zellen eines Patienten zu erhöhen, um so die positiven Eigenschaften dieser microRNA in Bezug auf ein spezifisches Krankheitsbild zu verstärken. Es kann auch dazu dienen, eine bestimmte microRNA, deren Gehalt bei einem bestimmten Krankheitsbild erhöht ist, durch Hybridisierung oder eine vergleichbare Interaktion zu eliminieren, um dem negativen Effekt dieser microRNA auf das entsprechende Krankheitsbild entgegenzuwirken.

Vorzugsweise stellt die ausgewählte Nukleinsäure oder Gruppe von Nukleinsäuren den einzigen pharmazeutisch aktiven Bestandteil des entsprechenden Medikaments dar.

Das Medikament eignet sich vorzugsweise nicht nur zur therapeutischen Zwecken, sondern auch zu diagnostischen Zwecken.

Bevorzugte Ausgestaltungen des oben dargestellten und nachfolgend in Zusammenhang mit den Beispielen erläuterten Verfahrens unter Verwendung der microRNAs sind in analoger Weise auch auf das beanspruchte Medikament übertragbar. Dies betrifft insbesondere die Auswahl entsprechender Nukleinsäure-Sequenzen oder Untergruppen von Sequenzen anhand der microRNAs, die als bevorzugt einzusetzen dargestellt sind.

Die Erfindung betrifft auch die therapeutische Nutzung der dargestellten microRNAs oder Nukleinsäure-Sequenzen, die zu diesen microRNAs identisch oder komplementär sind, insbesondere zur Behandlung bzw. Therapie von nicht-ischämischen Kardiomyopathien oder Speichererkrankungen des Herzens.

Bevorzugte Ausgestaltungen der oben dargestellten und nachfolgend in Zusammenhang mit den Beispielen erläuterten Verwendung der microRNAs sind in analoger Weise auch auf eine entsprechende therapeutische Anwendung übertragbar.

Die Erfindung betrifft auch ein in vivo durchgeführtes Diagnoseverfahren, dass in analoger Weise wie das vorliegend beschriebene In-vitro-Verfahren durchgeführt werden kann, wobei eine Entnahme einer Probe aus einem Patienten dazu nicht erforderlich ist.

Die vorliegende Erfindung soll anhand von Figuren und Beispielen näher erläutert werden. Es zeigen:
- Fig. 1A: eine Darstellung des Expressionsniveaus eines ersten microRNA-Markers in Abhängigkeit des jeweiligen Krankheitsbildes,
- Fig. 1B: eine Darstellung des Expressionsniveaus eines zweiten microRNA-Markers in Abhängigkeit des jeweiligen Krankheitsbildes,
- Fig. 1C: eine Darstellung des Expressionsniveaus eines dritten microRNA-Markers in Abhängigkeit des jeweiligen Krankheitsbildes,
- Fig. 2A: eine erste schematische Darstellung der diagnostischen Anwendung von Profilen zirkulierender microRNAs zur Erkennung und Differentialdiagnose biopsiepflichtiger Kardiomyopathie-Patienten und
- Fig. 2B: eine zweite schematische Darstellung der diagnostischen Anwendung von Profilen zirkulierender microRNAs zur Erkennung und Differentialdiagnose biopsiepflichtiger Kardiomyopathie-Patienten und

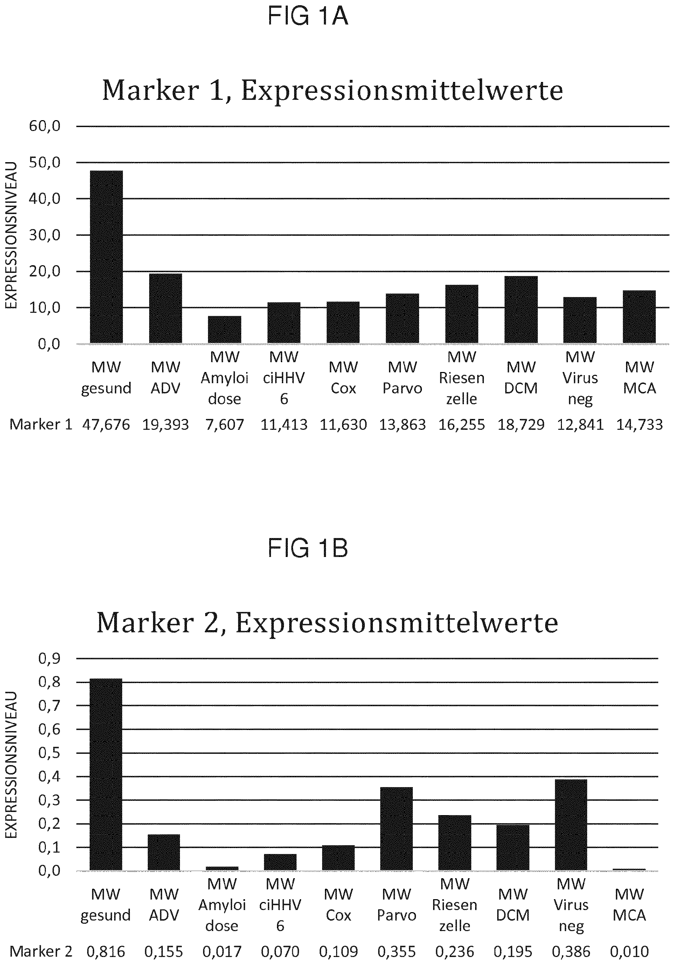

Die Figur 1A zeigt eine Darstellung der Expressionsmittelwerte eines ersten microRNA-Markers bei einer gesunden Kontrollgruppe und bei Patienten mit unterschiedlichen Krankheitsbildern. Dabei wurde als erster Marker die microRNA hsa-mir-197-3p (SEQ ID NO: 12) eingesetzt. Bei gesunden Patienten weist diese microRNA ein mittleres Expressionsniveau von knapp 50,0 auf. Bei Patienten mit einer Adenovirus-induzierten Kardiomyopathie (ADV) konnte ein Mittelwert (MW) von knapp 20 identifiziert werden. Bei Patienten mit einer Amyloidose des Herzens (Amyloidose), bei Patienten mit einer ciHHV6-induzierten Kardiomyopathie (ciHHV6), bei Patienten mit einer Coxsackievirus-induzierten Kardiomyopathie (Cox), bei Patienten mit einer Parvovirus-induzierten Kardiomyopathie (Parvo), bei Patienten mit einer Riesenzellenkardiomyopathie (Riesenzellen), bei Patienten mit einer dilatativen Kardiomyopathie (DCM), bei Patienten mit einer Kardiomyopathie ohne virale Entzündung des Herzens (Virus neg) und bei Patienten mit einer akuten Myokarditis (MCA) war das Expressionsniveau stets niedriger als bei den Patienten mit einer Adenovirus-induzierten Kardiomyopathie. Insgesamt zeigt sich durch die Figur 1A, dass die als Marker 1 eingesetzte microRNA ein guter Marker zur Unterscheidung von gesunden und kranken Patienten ist. Diese microRNA kann daher gut im Rahmen eines diagnostischen Profils als Marker zur Identifizierung von Patienten verwendet werden, bei denen keine Myokardbiopsie erforderlich ist.

Die Figur 1B zeigt eine Darstellung der Expressionsmittelwerte eines zweiten microRNA-Markers, nämlich der microRNA hsa-let-7f-5p (SEQ ID NO: 10). Auch bei dieser microRNA unterscheidet sich der Mittelwert des Expressionsniveaus bei gesunden Patienten signifikant vom Expressionsniveau bei Patienten, die an einer Kardiomyopathie leiden. Hinsichtlich der in der Figur 1B verwendeten Abkürzungen wird auf die Erläuterungen zur Figur 1A verwiesen. Damit ist auch die als Marker 2 eingesetzte microRNA ein guter Marker, um Patienten, bei denen keine Myokardbiopsie erforderlich ist, zu identifizieren.

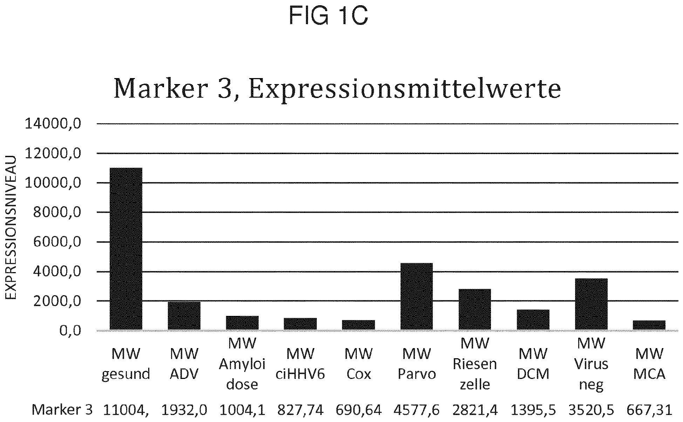

Die Figur 1C zeigt die Expressionsmittelwerte eines dritten Markers, nämlich der microRNA hsa-mir-223-3p (SEQ ID NO: 14). Auch bei dieser microRNA ist das Expressionsniveau bei gesunden Patienten signifikant höher als bei Patienten, die an einer spezifischen Kardiomyopathie leiden. Hinsichtlich der verwendeten Abkürzungen wird abermals auf die Erläuterungen zur Figur 1A verwiesen. Damit ist auch der Marker 3 ein geeigneter microRNA-Marker, um Patienten, bei denen keine Myokardbiopsie erforderlich ist, von Patienten, bei denen eine Myokardbiopsie zu weiteren diagnostischen Untersuchung erforderlich ist, zu unterscheiden.

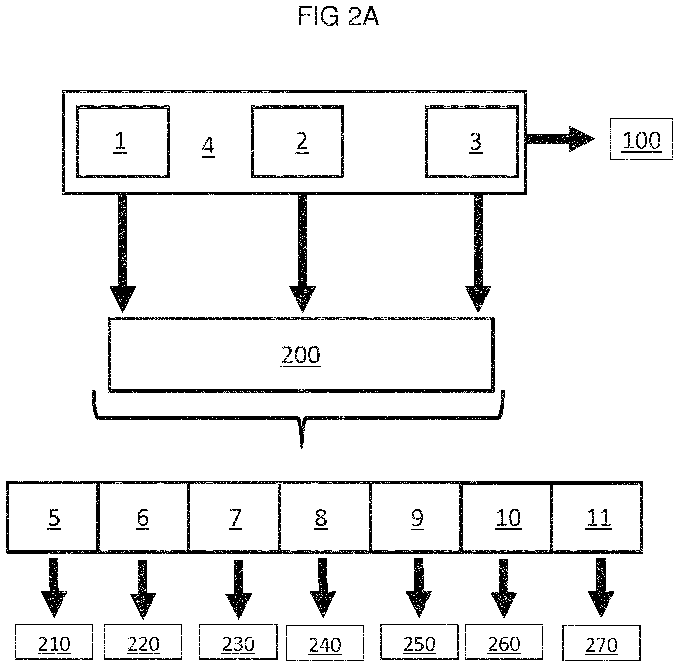

Die Figur 2A zeigt eine schematische Darstellung einer beispielhaften diagnostischen Anwendung von diagnostischen Profilen aus im Blutserum/Blutplasma zirkulierenden microRNAs zur Erkennung und Differentialdiagnose biopsiepflichtiger Kardiomyopathie-Patienten. Zunächst wird eine Blutserum-/Blutplasmaprobe eines Patienten auf das Vorhandensein eines ersten Markers 1, eines zweiten Markers 2 und eines dritten Markers 3 getestet. Bei diesen drei Markern kann es sich beispielsweise um die Marker handeln, deren Expressionsniveaus in den Figuren 1A bis 1C dargestellt sind.

Der erste Marker 1, der zweite Marker 2 und der dritte Marker 3 sind als diagnostisches Profil 4 zusammengefasst. Wenn ein hohes Expressionsniveau des ersten Markers 1, des zweiten Markers 2 und des dritten Markers 3 festgestellt wird, zeigt dies, dass der Patient gesund ist. In diesem Fall wird eine erste Diagnose 100 gestellt, gemäß der der Patient gesund ist und keine Myokardbiopsie erforderlich ist.

Ist hingegen das Expressionsniveau eines der drei Marker 1, 2, 3 nicht auf einem hohen Niveau (vergleiche hierzu die Figuren 1A bis 1C), wird eine zweite Diagnose 200 gestellt, gemäß der vermutlich eine Herzmuskelerkrankung vorliegt. Zur genaueren diagnostischen Identifikation der Herzmuskelerkrankung ist letztlich eine Myokardbiopsie erforderlich. Zur Erleichterung dieser Myokardbiopsie wird jedoch eine Vorklassifikation des untersuchten Patienten in eine Patientengruppe durchgeführt. Zu diesem Zweck wird die dem Patienten entnommene Probe mithilfe eines zweiten diagnostischen Profils 5, eines dritten diagnostischen Profils 6, eines vierten diagnostischen Profils 7, eines fünften diagnostischen Profils 8, eines sechsten diagnostischen Profils 9, eines siebten diagnostischen Profils 10 und eines achten diagnostischen Profils 11 näher analysiert.

Anhand des zweiten diagnostischen Profils 5 lässt sich feststellen, ob der Patient an einer Adenovirus-induzierten Kardiomyopathie leidet. Ist dies der Fall, wird eine entsprechende dritte Diagnose 210 erstellt. Bei der anschließenden Herzmuskelbiopsie kann dann gezielt auf die Anwesenheit von Adenoviren geschaut werden, was die entsprechende Diagnostik vereinfacht und das Diagnoseergebnis schneller verfügbar macht.

Mittels des dritten diagnostischen Profils 6 kann ermittelt werden, ob der Patient an einer Coxsackievirus-induzierten Kardiomyopathie leidet. Sofern dies der Fall ist, wird eine entsprechende vierte Diagnose 220 gestellt.

Mittels des vierten diagnostischen Profils 7 kann ermittelt werden, ob der Patient an einer Parvovirus-induzierten Kardiomyopathie leidet. Ist dies der Fall, wird eine entsprechende fünfte Diagnose 230 gestellt.

Mittels des fünften diagnostischen Profils 8 kann ermittelt werden, ob der Patient an einer (ci)HHV6-Virus-induzierten Kardiomyopathie leidet. Ist dies der Fall, wird eine entsprechende sechste Diagnose 240 gestellt.

Mittels des sechsten diagnostischen Profils 9 kann ermittelt werden, ob der Patient an einer inflammatorischen dilatativen Kardiomyopathie leidet. Ist dies der Fall, wird eine entsprechende siebte Diagnose 250 gestellt.

Mittels des siebten diagnostischen Profils 10 kann ermittelt werden, ob der Patient an einer kardialen Sarkoidose bzw. an einer Riesenzellenmyokarditis leitet. Ist dies der Fall, wird eine entsprechende achte Diagnose 260 erstellt.

Mittels des achten diagnostischen Profils 11 kann ermittelt werden, ob der Patient an einer Amyloidose des Herzens leidet. Ist dies der Fall, wird eine entsprechende neunte Diagnose 270 erstellt.

Jedes der diagnostischen Profile 4, 5, 6, 7, 8, 9, 10, 11 weist mindestens drei microRNAs als Marker auf. Es ist jedoch möglich, dass die einzelnen diagnostischen Profile noch mehr microRNAs umfassen.

Es ist möglich, die zu untersuchende Probe gleichzeitig mittels aller diagnostischen Profile 4, 5, 6, 7, 8, 9, 10, 11 zu analysieren. Es kann dann eine sequenzielle Auswertung erfolgen, ob der Patient gesund oder krank ist und wenn er krank ist, an welcher Kardiomyopathieform er erkrankt ist.

Alternativ wäre es auch möglich, zunächst nur ein diagnostisches Profil zur Analyse der Probe des Patienten einzusetzen und in Abhängigkeit des Analyseergebnisses nachfolgend weitere Analyseschritte mit weiteren diagnostischen Profilen vorzunehmen. Dies ist vom Arbeitsablauf und von der Probenhandhabung jedoch häufig aufwendiger als eine gleichzeitige Analyse aller diagnostischen Profile.

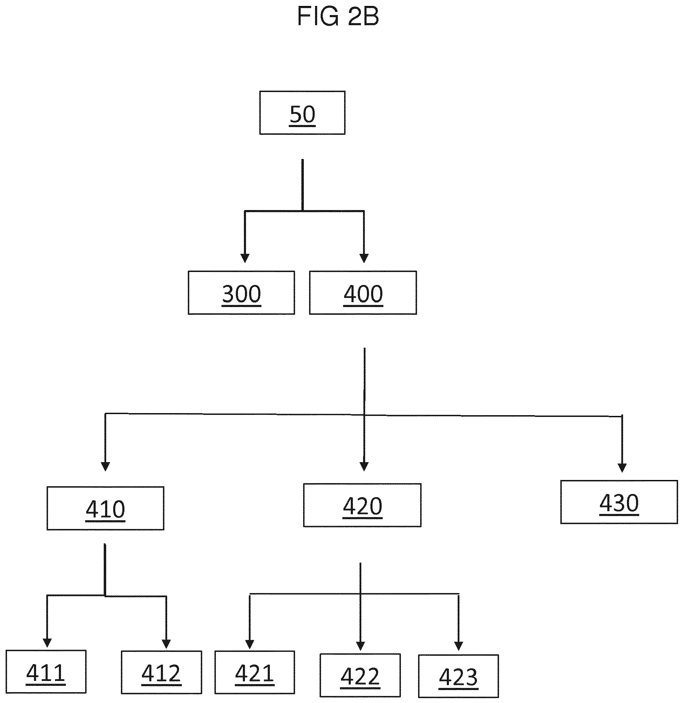

Die Figur 2B zeigt eine zweite schematische Darstellung einer möglichen diagnostischen Anwendung von diagnostischen Profilen aus im Blutserum/Blutplasma zirkulierenden microRNAs zur Erkennung und Differentialdiagnose biopsiepflichtiger Kardiomyopathie-Patienten.

Zunächst wird eine Probe 50 eines Patienten bereitgestellt, wobei nicht bekannt ist, ob der Patient gesund ist oder an einer Kardiomyopathie erkrankt ist. In einem ersten Diagnoseschritt wird daher entweder eine erste Diagnose 300 "Patient gesund" oder eine zweite Diagnose 400 "Patient krank" gestellt.

Ist der Patient gesund, bedarf es keiner weiteren Untersuchung der Probe 50 des Patienten. Ist der Patient hingegen krank, wird die Probe 50 des Patienten näher analysiert, um Aufschluss über die Art der Kardiomyopathie zu erhalten, an der der Patient erkrankt ist. Im Rahmen dieses zweiten Diagnoseschritts kann dann beispielsweise eine dritte Diagnose 410 gestellt werden, gemäß der der Patient an einer Amyloidose des Herzens erkrankt ist. Alternativ kann im zweiten Diagnoseschritt auch eine vierte Diagnose 420 erstellt werden, gemäß der der Patient an einer Virus-induzierten Kardiomyopathie erkrankt ist. Schließlich kann in diesem zweiten Diagnoseschritt auch eine fünfte Diagnose 430 erstellt werden, gemäß der der Patient an einer inflammatorischen dilatativen Kardiomyopathie erkrankt ist.

Nun kann in einem weiteren Diagnoseschritt eine Subklassifizierung der zuvor ermittelten Krankheitsbilder erfolgen. Wurde beispielsweise durch die dritte Diagnose 410 ermittelt, dass der Patient an einer Amyloidose des Herzens erkrankt ist, kann in einem dritten Diagnoseschritt ermittelt werden, um welche Art der Amyloidose sich dabei handelt. Im Rahmen des dritten Diagnoseschritts kann dabei eine sechste Diagnose 411 erstellt werden, gemäß der der Patient an einer ATTR-Amyloidose erkrankt ist. Alternativ kann im dritten Diagnoseschritt auch eine siebte Diagnose 412 erstellt werden, gemäß der der Patient an einer Lambda-Amyloidose erkrankt ist.

Hat sich im zweiten Diagnoseschritt durch die vierte Diagnose 420 gezeigt, dass der Patient an einer Virus-induzierten Kardiomyopathie erkrankt ist, kann im dritten Diagnoseschritt durch eine achte Diagnose 421 festgestellt werden, dass der Patient an einer Enterovirus-induzierten Kardiomyopathie erkrankt ist. Alternativ kann durch eine neunte Diagnose 422 festgestellt werden, dass der Patient an einer (ci)HHV6-induzierten Kardiomyopathie erkrankt ist. Schließlich kann im dritten Diagnoseschritt durch eine zehnte Diagnose 423 festgestellt werden, dass der Patient an einer Coxsackievirus-induzierten Kardiomyopathie erkrankt ist.

Der erste Diagnoseschritt, der zweite Diagnoseschritt und der dritte Diagnoseschritt können - wie bereits in Zusammenhang mit der Darstellung der Figur 2 A erläutert - gleichzeitig durch Anwendung unterschiedlicher diagnostischer Profile ausgeführt werden.

### Ausführungsbeispiele

Um im Rahmen der nachfolgend erläuterten Beispiele angeben zu können, ob die Expression der betrachteten microRNAs herauf reguliert oder herunter reguliert ist, wurden diejenigen microRNAs identifiziert, die in über 95 % aller untersuchten Blutserum-/Blutplasmaproben relativ konstant exprimiert waren. Hierzu wurde entsprechend der in Zusammenhang mit Beispiel 1 beschriebenen Vorgehensweise vorgegangen. Dabei wurden sowohl kardiologisch gesunde Blutspender (Beispiel 1) als auch Patienten mit durch Myokardbiopsien diagnostizierbaren Kardiomyopathien viraler-entzündlicher Genese (Beispiele 2 bis 8) berücksichtigt. Diese konstitutiv exprimierten microRNAs werden auch als HaushaltsmicroRNAs oder Housekeeping-microRNAs bezeichnet. Diese Housekeeping-microRNAs wurden dann als Normalisierungssequenzen verwendet, um relative Aussagen zur Expressionsmodifikation der anderen untersuchten microRNAs treffen zu können.

Die identifizierten Housekeeping-microRNAs sind in der nachfolgenden Tabelle 1 aufgelistet. Die Expression von krankheitsrelevanten microRNAs laut Beispielen 2 bis 8 wurden gegen ein oder mehrere Housekeeping-microRNAs normalisiert, um das diagnose-spezifische Expressionsniveau festzustellen (vergleiche hierzu beispielsweise Figur 1).

**Tabelle 1: Konstitutiv exprimierte microRNAs (Housekeeping-microRNAs) als Normalisierungssequenzen für die Messung differentiell regulierter microRNAs in Blutserum-/Blutplasmaproben**

| **SEQ ID NO** | **microRNA** | **Regulation ↑↓** |
|---|---|---|
| 1 | hsa-miR-10a-5p | HK |
| 2 | hsa-miR-10b-5p | HK |
| 3 | hsa-miR-130b-3p | HK |
| 4 | hsa-miR-148b-3p | HK |
| 5 | hsa-miR-152-3p | HK |
| 6 | hsa-miR-185-5p | HK |
| 7 | hsa-miR-27b-3p | HK |
| 8 | hsa-miR-30a-3p | HK |
| 9 | hsa-miR-335-5p | HK |

| | | |
|---|---|---|
| HK = Housekeeping | | |

### Beispiel 1: Diagnose von Patienten ohne Verdacht auf eine viral-entzündliche Herzmuskelerkrankung oder auf eine Amyloidose mittels einer Blutserum/Blutplasmaprobe (Identifikation nicht biopsiepflichtiaer Patienten)

Mehreren menschlichen Patienten, die an keiner der bisher durch Myokardbiopsien diagnostizierbaren Kardiomyopathien viral-entzündlicher Genese erkrankt waren bzw. deren Symptome noch nicht diagnostizierbar ausgeprägt waren (Kontrollgruppe), wurde eine Blutserum-/Blutplasmaprobe entnommen.

Aus den einzelnen Proben wurde mittels dem Fachmann allgemein bekannter Standardverfahren jeweils ein Total-RNA-Extrakt (also ein Extrakt der gesamten RNA der jeweiligen Blutserum-/Blutplasmaprobe) gewonnen, welcher auch microRNA-Bestandteile enthielt. Dieser Total-RNA-Extrakt wurde anschließend mittels zweier Varianten auf das Vorhandensein bestimmter microRNAs untersucht.

### Variante 1 (RNA-Chip/Mikropartikel)

Die RNA des Total-RNA-Extrakts wurde mit Biotin markiert, auf einen RNA-Chip aufgebracht und 16 Stunden inkubiert. Ein solcher RNA-Chip wird mitunter auch als DNA-Chip bezeichnet, da er DNA-Sonden enthält. Diese sind jedoch zum RNA-Nachweis vorgesehen, weshalb vorliegend die Bezeichnung "RNA-Chip" verwendet wird. Während der Inkubation erfolgte eine Hybridisierung der in dem Total-RNA-Extrakt enthaltenen microRNA mit komplementären DNA-Sonden auf dem RNA-Chip. Hybridisierte microRNA-Moleküle wurden anschließend mit dem Fluoreszenzfarbstoff Streptavidin-Phycoerythrin, welcher an Biotin bindet, detektiert. Durch Bestimmung der Fluoreszenzintensität des gebundenen Streptavidin-Phycoerythrins konnte die Menge der hybridisierten microRNA in semi-quantitativer Weise bestimmt werden. Analog kann die Hybridisierung der gesuchten microRNAs auch an verschiedenfarbige Mikropartikel erfolgen, welche jeweils eine oder mehrere komplementäre DNA- oder RNA-Sonden an ihrer Oberfläche tragen. Nach dem Bindungs-, Wasch- und Markierungsschritten kann dann ein Nachweis mit mikroskopischen oder durchflusszytometrischen Verfahren erfolgen.

Für die einzelnen Verfahrensschritte wurden vom jeweiligen Hersteller der RNA-Chips empfohlene Standardpuffer oder eigens hergestellte Lösungen verwendet. Diese sind dem Fachmann allgemein geläufig. Für den direkten microRNA-Nachweis wurden "Quantigene"-Kits der Fa. Affymetrix und für die den durchflusszytometrischen Nachweis Mikropartikel und Kits der Firma Luminex verwendet.

### Variante 2 (PCR-Genkarte)

Die im Total-RNA-Extrakt enthaltene microRNA wurde zur Synthese von cDNA verwendet. Je nach Menge der erhaltenen cDNA wurde diese bei Bedarf präamplifiziert. Anschließend wurde die cDNA auf eine Karte aufgetragen und eine quantitative PCR durchgeführt. Auf diese Weise konnte eine relative Quantifizierung der einzelnen cDNAs in Echtzeit über die relative Fluoreszenz zu konstitutiv exprimierten microRNAs erfolgen.

Für die einzelnen Verfahrensschritte wurden vom jeweiligen Anbieter der Genkarten empfohlene Standardpuffer verwendet. Diese sind dem Fachmann allgemein geläufig. Unter anderem wurden Genkarten der Firma Applied Biosystems verwendet.

Durch eine ergänzende Anwendung der Verfahren nach Variante 1 und Variante 2 wurden zahlreiche microRNAs identifiziert, deren Expression im Vergleich zu Proben, die von Patienten mit nicht-ischämischen Kardiomyopathien oder einer Speichererkrankung des Herzens stammten, modifiziert war.

In der nachfolgenden Tabelle 2 sind die identifizierten microRNAs, deren Expression bei der Kontrollgruppe im Vergleich zu untersuchten Proben von Kardiomyopathie-Patienten modifiziert ist, aufgelistet. Ferner ist in dieser Tabelle angegeben, welcher Art diese Modifikation ist. Dazu wurde unter Berücksichtigung der in den nachfolgenden Beispielen erläuterten Ergebnissen ein Punktwert berechnet und ermittelt, ob die Expression der jeweiligen microRNA hoch oder herunter reguliert war. Die genaue Bedeutung des Punktwerts wird in Zusammenhang mit Beispiel 2 erläutert.

Ferner wurde ein Signifikanzwert berechnet, der die Signifikanz der jeweiligen microRNA für die betrachtete Fragestellung angibt. In den Signifikanzwert fließen die Ausprägung der Expressionsmodifikation, die Nachweisbarkeit der jeweiligen microRNA auf unterschiedlichen Nachweisplattformen, der Punktwert sowie weitere Parameter ein. Der Signifikanzwert kann Werte von 1 bis 6 annehmen, wobei 1 eine hohe Signifikanz bedeutet und 6 eine niedrige Signifikanz.

Die identifizierten microRNAs aus Tabelle 2 sind besonders geeignet, Patienten zu erkennen, welche zum Untersuchungszeitpunkt keine Biopsieuntersuchung benötigen bzw. die Entscheidung für eine Biopsienentnahme zu einem zeitlich späteren Untersuchungszeitpunkt gemacht werden kann, sollten sich die klinischen Parameter oder das Muster der zirkulierenden microRNAs verändern.

Die identifizierten microRNAs aus Tabelle 2 sind auch besonders geeignet für eine sequentielle Differentialdiagnose entsprechend der in den Figuren 2A und 2B beispielhaft beschriebenen Vorgehensweise geeignet, um die biopsiepflichtigen Patienten zu erkennen und diese anschließend einer mögliche Subklassen-Analyse zu unterziehen.

**Tabelle 2: Expressionsmodifikationen von microRNAs zur Diagnose derzeit nicht biopsiepflichtiger Patienten für das Krankheitsbild viral-entzündlicher Kardiomyopathien.**

| **SEQ ID NO** | **microRNA** | **Regulation ↑↓** | **Signifikanzwert** | **Punktwert** |
|---|---|---|---|---|
| 10 | hsa-let-7f-5p | hoch | 1 | 1 |
| 11 | hsa-miR-103a-3p | hoch | 4 | 2 |
| 12 | hsa-miR-197-3p | hoch | 1 | 1 |
| 13 | hsa-miR-215-5p | hoch | 2 | 4 |
| 14 | hsa-miR-223-3p | hoch | 1 | 1 |
| 15 | hsa-miR-23a-3p | hoch | 1 | 1 |
| 16 | hsa-miR-330-3p | hoch | 3 | 1 |
| 17 | hsa-miR-337-5p | hoch | 3 | 1 |
| 18 | hsa-miR-339-3p | hoch | 3 | 1 |
| 19 | hsa-miR-339-5p | hoch | 3 | 1 |
| 20 | hsa-miR-411-5p | herunter | 4 | 3 |
| 21 | hsa-miR-454-3p | hoch | 3 | 1 |
| 22 | hsa-miR-485-3p | hoch | 5 | 3 |
| 23 | hsa-miR-487b-3p | hoch | 5 | 3 |
| 24 | hsa-miR-494-3p | hoch | 5 | 3 |
| 25 | hsa-miR-505-3p | hoch | 3 | 1 |
| 26 | hsa-miR-628-5p | hoch | 5 | 3 |
| 27 | hsa-miR-671-3p | hoch | 5 | 3 |
| 28 | hsa-miR-744-5p | hoch | 3 | 1 |
| 29 | hsa-miR-889-3p | hoch | 5 | 3 |
| 30 | mmu-miR-495-3p | hoch | 3 | 1 |

In einer Variante bezieht sich die Erfindung auf die Verwendung der in der Tabelle 2 mit einem Punktwert von 1 und/oder 2 und/oder einem Signifikanzwert von 1 und/oder 2 versehenen microRNAs einzeln oder in beliebiger Kombination miteinander als Marker zur Identifizierung nicht-biopsiepflichtiger Patienten. Eine Kombination eines Punktwerts von 1 mit einem Signifikanzwert von 1 zeigt dabei besonders geeignete microRNAs an.

### Beispiel 2: Diagnose von Adenovirus-induzierten Kardiomyopathien mittels einer mittels einer Blutserum-/Blutplasmaprobe

Es wurde analog zu Beispiel 1 vorgegangen, nur dass die Blutserum-/Blutplasmaproben Patienten entnommen wurden, die an einer Adenovirus-induzierten Kardiomyopathie erkrankten waren.

In der nachfolgenden Tabelle 3 sind die identifizierten microRNAs, deren Expression bei einer Adenovirus-induzierten Kardiomyopathie modifiziert ist, aufgelistet. Ferner ist in dieser Tabelle angegeben, welcher Art diese Modifikation ist. Dazu wurde unter Berücksichtigung der in den Beispielen erläuterten Ergebnissen abermals ein Punktwert berechnet und ermittelt, ob die Expression der jeweiligen microRNA hoch oder herunter reguliert war.

Ein Punktwert von 1 bedeutet, dass die beobachtete Expressionsmodifikation spezifisch für eine der betrachteten nicht-ischämischen Kardiomyopathien oder Speichererkrankungen des Herzens ist und nur bei dieser deutlich verändert auftritt. MicroRNAs, denen ein Punktwert von 1 zugeordnet ist, sind daher spezifische Marker für eine Adenovirus-induzierte Kardiomyopathie. Ihre Markerfunktion können diese microRNAs durch eine erhöhte Expression (Hochregulation) oder eine verminderte Expression (Herunterregulation) ausüben. Ein Punktwert von 1 bezeichnet mit anderen Worten microRNAs, die eine eindeutige Identifizierung eines Krankheitsbildes ermöglichen, selbst wenn nicht bekannt ist, welcher Art die beobachtete Modifikation der Expression ist.

Ein Punktwert von 2 bedeutet, dass die beobachtete Expressionsmodifikation spezifisch für zwei der betrachteten nicht-ischämischen Kardiomyopathien oder Speichererkrankungen des Herzens ist und nur bei diesen beiden Erkrankungen auftritt. Dabei ist die Expression im Falle der ersten dieser beiden Erkrankungen erhöht und im Falle der zweiten dieser beiden Erkrankungen erniedrigt. MicroRNAs, denen ein Punktwert von 2 zugeordnet ist, sind daher spezifische Marker für eine Adenovirus-induzierte Kardiomyopathie, sofern zusätzlich bekannt ist, ob ihre Expression hochreguliert oder aber herunterreguliert ist. Ein Punktwert von 2 bezeichnet mit anderen Worten microRNAs, die eine eindeutige Identifizierung eines Krankheitsbildes ermöglichen, sofern zusätzlich bekannt ist, welcher Art die beobachtete Modifikation der Expression ist.

Ein Punktwert von 3 bedeutet, dass die beobachtete Expressionsmodifikation signifikant für mehrere der betrachteten nicht-ischämischen Kardiomyopathien oder Speichererkrankungen des Herzens ist. Dabei ist die Expression im Falle zweier Erkrankungen gleichläufig modifiziert, also entweder erhöht oder erniedrigt. Bei allen anderen Erkrankungen, bei denen die Expressionsmodifikation ebenfalls signifikant ist, ist die Expression gegenläufig modifiziert, also entweder erniedrigt oder erhöht. MicroRNAs, denen ein Punktwert von 3 zugeordnet ist, sind daher für sich allein genommen unspezifische Marker für eine Adenovirus-induzierte Kardiomyopathie, da sie auch ein anderes Krankheitsbild anzeigen könnten. Zusammen mit einem weiteren microRNA-Marker, der entweder spezifisch oder aber ebenfalls unspezifisch ist, lässt sich jedoch bereits eine eindeutige Bestimmung einer Adenovirus-induzierten Kardiomyopathie ermöglichen. Dies gilt im Falle eines weiteren unspezifischen Markers jedoch nur, sofern dessen Expression bei anderen weiteren Erkrankungen modifiziert ist als denjenigen weiteren Erkrankungen, bei denen die Expression des ersten unspezifischen Markers modifiziert ist.

Ein Punktwert von 4 bedeutet, dass die beobachtete Expressionsmodifikation signifikant für genau zwei der betrachteten nicht-ischämischen Kardiomyopathien oder Speichererkrankungen des Herzens ist. Dabei ist die Expression in beiden Fällen gleichläufig modifiziert, also entweder erhöht oder erniedrigt. MicroRNAs, denen ein Punktwert von 4 zugeordnet ist, sind daher für sich allein genommen unspezifische Marker für eine Adenovirus-induzierte Kardiomyopathie, da sie auch ein anderes Krankheitsbild anzeigen könnten. Zusammen mit einem weiteren microRNA-Marker, der entweder spezifisch oder aber ebenfalls unspezifisch ist, lässt sich jedoch bereits eine eindeutige Bestimmung einer Adenovirus-induzierten Kardiomyopathie ermöglichen. Dies gilt im Falle eines weiteren unspezifischen Markers jedoch nur, sofern dessen Expression bei einer anderen weiteren Erkrankung modifiziert ist als derjenigen weiteren Erkrankung, bei der die Expression des ersten unspezifischen Markers gleichermaßen modifiziert ist.

Hinsichtlich des ebenfalls ermittelten Signifikanzwerts wird auf die obigen Erläuterungen verwiesen.

**Tabelle 3: Expressionsmodifikationen von microRNAs bei einer Adenovirus-induzierten Kardiomyopathie.**

| **SEQ ID NO** | **microRNA** | **Regulation ↑↓** | **Signifikanzwert** | **Punktwert** |
|---|---|---|---|---|
| 31 | hsa-miR-1274B | herunter | 6 | 4 |
| 32 | hsa-miR-136-3p | hoch | 3 | 1 |
| 33 | hsa-miR-187-3p | herunter | 4 | 1 |
| 34 | hsa-miR-199a-5p | hoch | 5 | 3 |
| 35 | hsa-miR-215-5p | herunter | 2 | 4 |
| 36 | hsa-mir-422a | herunter | 4 | 4 |
| 37 | hsa-miR-4449 | hoch | 3 | 1 |
| 38 | hsa-miR-4674 | hoch | 3 | 1 |
| 39 | hsa-miR-5096 | hoch | 3 | 1 |
| 40 | hsa-miR-548c-3p | hoch | 5 | 3 |
| 41 | hsa-miR-548c-5p | hoch | 6 | 3 |
| 42 | hsa-miR-650 | hoch | 1 | 1 |

In einer Variante bezieht sich die Erfindung auf die Verwendung der in der Tabelle 3 mit einem Punktwert von 1 und/oder 2 und/oder einem Signifikanzwert von 1 und/oder 2 versehenen microRNAs einzeln oder in beliebiger Kombination miteinander als Marker zur Identifizierung einer Adenovirus-induzierten Kardiomyopathie. Eine Kombination eines Punktwerts von 1 mit einem Signifikanzwert von 1 zeigt dabei besonders geeignete microRNAs an.

### Beispiel 3: Diagnose von Enterovirus-induzierten (Coxsackievirus-induzierten) Kardiomyopathien mittels einer Blutserum-/Blutplasmaprobe

Es wurde analog zu Beispiel 1 vorgegangen, nur dass die Blutserum-/Blutplasmaproben Patienten entnommen wurden, die an einer Enterovirus-(Coxsackievirus-)induzierten Kardiomyopathie erkrankt waren. Die identifizierten microRNAs, deren Expression bei einer solchen Enterovirus-(Coxsackievirus-)induzierten Kardiomyopathie modifiziert sind, sind in der nachfolgenden

Tabelle **4** aufgelistet. Hinsichtlich der Punktwerte und Signifikanzwerte wird auf die Erläuterungen zu den Beispielen 1 und 2 verwiesen.

**Tabelle 4: Expressionsmodifikationen von microRNAs bei einer Enterovirus-(Coxsackievirus-)induzierten Kardiomyopathie.**

| **SEQ ID NO** | **microRNA** | **Regulation ↑↓** | **Signifikanzwert** | **Punktwert** |
|---|---|---|---|---|
| 43 | hsa-miR-1180-3p | herunter | 3 | 1 |
| 44 | hsa-miR-1-3p | hoch | 3 | 1 |
| 45 | hsa-miR-140-5p | hoch | 3 | 1 |
| 46 | hsa-miR-150-3p | hoch | 3 | 1 |
| 47 | hsa-miR-16-5p | herunter | 4 | 1 |
| 48 | hsa-miR-215-5p | herunter | 2 | 4 |
| 49 | hsa-miR-25-3p | herunter | 1 | 1 |
| 50 | hsa-miR-30a-5p | herunter | 5 | 3 |
| 51 | hsa-miR-30e-5p | hoch | 3 | 1 |
| 52 | hsa-miR-3138 | hoch | 3 | 1 |
| 53 | hsa-miR-3622b-5p | hoch | 3 | 1 |
| 54 | hsa-miR-3922-5p | hoch | 5 | 2 |
| 55 | hsa-mir-422a | hoch | 4 | 4 |
| 56 | hsa-miR-4274 | herunter | 3 | 1 |
| 57 | hsa-miR-4303 | herunter | 3 | 1 |
| 58 | hsa-miR-4419b | herunter | 3 | 1 |
| 59 | hsa-miR-4429 | hoch | 5 | 3 |
| 60 | hsa-miR-4467 | herunter | 3 | 1 |
| 61 | hsa-miR-4651 | hoch | 3 | 1 |
| 62 | hsa-miR-4716-5p | hoch | 3 | 1 |
| 63 | hsa-miR-548b-5p | herunter | 3 | 1 |
| 64 | hsa-miR-548c-3p | hoch | 5 | 3 |
| 65 | hsa-miR-597-5p | hoch | 5 | 3 |
| 66 | hsa-miR-601 | hoch | 4 | 3 |
| 67 | hsa-miR-629-3p | herunter | 3 | 1 |
| 68 | hsa-miR-660-5p | herunter | 6 | 3 |
| 69 | hsa-miR-886-3p | hoch | 5 | 3 |
| 70 | hsa-miR-923 | hoch | 3 | 1 |
| 71 | mmu-miR-374-5p | hoch | 6 | 3 |

In einer Variante bezieht sich die Erfindung auf die Verwendung der in der

Tabelle **4** mit einem Punktwert von 1 und/oder 2 und/oder 2 und/oder einem Signifikanzwert von 1 und/oder 2 versehenen microRNAs einzeln oder in beliebiger Kombination miteinander als Marker zur Identifizierung einer Enterovirus-(Coxsackievirus-)induzierten Kardiomyopathie. Eine Kombination eines Punktwerts von 1 mit einem Signifikanzwert von 1 zeigt dabei besonders geeignete microRNAs an.

### Beispiel 4: Diagnose einer Amyloidose des Herzens mittels einer Blutserum-/Blutplasmaprobe

Es wurde analog zu Beispiel 1 vorgegangen, nur dass die Blutserum-/Blutplasmaproben Patienten entnommen wurden, die an einer Amyloidose des Herzens erkrankt waren.

Eine Amyloidose ist eine typische Speichererkrankung des Herzens und ist durch Amyloidablagerungen im Herzmuskel gekennzeichnet.

Die identifizierten microRNAs, deren Expression bei einer solchen Amyloidose modifiziert sind, sind in der nachfolgenden Tabelle 5 aufgelistet. Hinsichtlich der Punktwerte und Signifikanzwerte wird auf die Erläuterungen zu den Beispielen 1 und 2 verwiesen.

Einige der microRNAs, die in der Tabelle 5 enthalten sind, eignen sich zudem zur Subklassifizierung von zwei der häufigsten Formen kardialer Amyloidose (Lambda-Amyloidose und ATTR-Amyloidose). Die zur Subklassifizierung geeigneten microRNAs sin den nachfolgenden Tabellen 6 und 7 aufgelistet.

**Tabelle 5: Expressionsmodifikationen von microRNAs bei einer Amyloidose des Herzens.**

| **SEQ ID NO** | **microRNA** | **Regulation ↑↓** | **Signifikanzwert** | **Punktwert** |
|---|---|---|---|---|
| 72 | hsa-miR-103a-3p | hoch | 4 | 2 |
| 73 | hsa-miR-106b-5p | herunter | 3 | 1 |
| 74 | hsa-miR-126-3p | herunter | 3 | 1 |
| 75 | hsa-miR-126-5p | herunter | 3 | 1 |
| 76 | hsa-miR-1274A | herunter | 4 | 1 |
| 77 | hsa-miR-1274B | herunter | 6 | 4 |
| 78 | hsa-miR-132-3p | hoch | 3 | 1 |
| 79 | hsa-miR-140-3p | herunter | 4 | 1 |
| 80 | hsa-miR-142-3p | herunter | 3 | 1 |
| 81 | hsa-miR-146b-5p | herunter | 3 | 1 |
| 82 | hsa-miR-150-5p | herunter | 3 | 1 |
| 83 | hsa-miR-15b-5p | herunter | 3 | 1 |
| 84 | hsa-miR-181b-5p | herunter | 3 | 1 |
| 85 | hsa-miR-186-5p | herunter | 3 | 1 |
| 86 | hsa-miR-21-5p | herunter | 3 | 1 |
| 87 | hsa-miR-222-3p | herunter | 4 | 1 |
| 88 | hsa-miR-223-5p | herunter | 3 | 1 |
| 89 | hsa-miR-24-3p | herunter | 4 | 3 |
| 90 | hsa-miR-26b-5p | herunter | 3 | 1 |
| 91 | hsa-miR-30a-3p | herunter | 1 | 1 |
| 92 | hsa-miR-30d-5p | herunter | 3 | 1 |
| 93 | hsa-miR-320a | herunter | 3 | 1 |
| 94 | hsa-miR-320c | hoch | 5 | 3 |
| 95 | hsa-miR-328-3p | herunter | 3 | 1 |
| 96 | hsa-miR-375 | hoch | 3 | 1 |
| 97 | hsa-miR-378-5p | herunter | 3 | 1 |
| 98 | hsa-mir-423 | hoch | 3 | 1 |
| 99 | hsa-miR-4286 | herunter | 3 | 1 |
| 100 | hsa-miR-451a | herunter | 3 | 1 |
| 101 | hsa-miR-4535 | hoch | 4 | 3 |
| 102 | hsa-miR-483-5p | herunter | 5 | 3 |
| 103 | hsa-miR-518d-3p | hoch | 6 | 3 |
| 104 | hsa-miR-532-5p | herunter | 3 | 1 |
| 105 | hsa-miR-590-5p | herunter | 5 | 3 |
| 106 | hsa-miR-601 | hoch | 4 | 3 |
| 107 | hsa-miR-660-5p | herunter | 6 | 3 |
| 108 | hsa-miR-885-5p | herunter | 3 | 1 |
| 109 | hsa-miR-92a-3p | herunter | 6 | 3 |
| 110 | hsa-miR-99b-5p | herunter | 3 | 1 |

**Tabelle 6: Zur Subklassifizierung der Lambda-Amyloidose geeignete microRNAs**

| **SEQ ID NO** | **microRNA** | **Regulation ↑↓** | **Signifikanzwert** | **Punktwert** |
|---|---|---|---|---|
| 73 | hsa-miR-106b-5p | herunter | 3 | 1 |
| 76 | hsa-miR-1274A | herunter | 4 | 1 |
| 91 | hsa-miR-30a-3p | herunter | 1 | 1 |
| 101 | hsa-miR-4535 | hoch | 4 | 3 |

**Tabelle 7: Zur Subklassifizierung der ATTR-Amyloidose geeignete microRNAs**

| **SEQ ID NO** | **microRNA** | **Regulation ↑↓** | **Signifikanzwert** | **Punktwert** |
|---|---|---|---|---|
| 79 | hsa-miR-140-3p | herunter | 4 | 1 |
| 84 | hsa-miR-181b-5p | herunter | 3 | 1 |
| 87 | hsa-miR-222-3p | herunter | 4 | 1 |
| 88 | hsa-miR-223-5p | herunter | 3 | 1 |

In einer Variante bezieht sich die Erfindung auf die Verwendung der in den Tabellen 5 bis 7 mit einem Punktwert von 1 und/oder 2 und/oder 2 und/oder einem Signifikanzwert von 1 und/oder 2 versehenen microRNAs einzeln oder in beliebiger Kombination miteinander als Marker zur Identifizierung einer Speichererkrankung des Herzens, insbesondere einer Amyloidose des Herzens. Eine Kombination eines Punktwerts von 1 mit einem Signifikanzwert von 1 zeigt dabei besonders geeignete microRNAs an.

### Beispiel 5: Diagnose einer kardialen Sarkoidose (CS) / Riesenzellmvokarditis mittels einer Blutserum-/Blutplasmaprobe

Es wurde analog zu Beispiel 1 vorgegangen, nur dass die Blutserum-/Blutplasmaproben Patienten entnommen wurden, die an einer Riesenzellmyokarditis erkrankt waren.

Eine kardiale Sarkoidose oder Riesenzellmyokarditis ist durch das Auftreten von mehrkernigen Riesenzellen oft begleitend zu einer akuten Myokarditis im Herzmuskel gekennzeichnet. Da unbehandelte Myokarditiden mit Riesenzellen meist einen fatalen Verlauf nehmen, muss diese Form der akuten Myokarditiden genau bestimmt werden, was bisher nur durch histologische Begutachtung von Paraffinschnitten möglich ist. Allerdings gelingt der Nachweis der mehrkernigen Riesenzellen nur extrem selten. In Verdachtsfällen müssen oft 10 und mehr Herzmuskelbiopsien aufgeschnitten werden, damit ein histologischer Beleg gefunden werden kann, was fast kein kardiologisches Zentrum weltweit durchführt.

Die identifizierten microRNAs, deren Expression bei einer solchen Myokarditis mit myokardialen Riesenzellen modifiziert sind, sind in der nachfolgenden Tabelle 8 aufgelistet. Hinsichtlich der Punktwerte und Signifikanzwerte wird auf die Erläuterungen zu den Beispielen 1 und 2 verwiesen.

**Tabelle 8: Expressionsmodifikationen von microRNAs bei einer kardialen Sarkoidose/Riesenzellmyokarditis.**

| **SEQ ID NO** | **microRNA** | **Regulation ↑↓** | **Signifikanzwert** | **Punktwert** |
|---|---|---|---|---|
| 111 | hsa-miR-139-3p | hoch | 5 | 3 |
| 112 | hsa-miR-210-3p | hoch | 3 | 1 |
| 113 | hsa-miR-296-5p | hoch | 3 | 1 |
| 114 | hsa-miR-486-3p | hoch | 5 | 3 |
| 115 | hsa-miR-500a-5p | hoch | 3 | 1 |
| 116 | hsa-miR-542-3p | hoch | 3 | 1 |

In einer Variante bezieht sich die Erfindung auf die Verwendung der in der Tabelle 8 mit einem Punktwert von 1 und/oder 2 und/oder 2 und/oder einem Signifikanzwert von 1 und/oder 2versehenen microRNAs einzeln oder in beliebiger Kombination miteinander als Marker zur Identifizierung einer Riesenzellmyokarditis.

### Beispiel 6: Diagnose einer Ervthrovirus-induzierten Kardiomyopathie mittels einer Blutserum-/Blutplasmaprobe

Es wurde analog zu Beispiel 1 vorgegangen, nur dass die Blutserum-/Blutplasmaproben Patienten entnommen wurden, die an einer Erythrovirus-induzierten Kardiomyopathie erkrankt waren.

Das Erythrovirus, das eine derartige Kardiomyopathie verursacht, tritt in zwei Subtypen auf, wobei der Genotyp 1 als Parvovirus B19 bzw. Erythrovirus B19 bekannt ist. Bei Myokardinfektionen spielt das Parvovirus jedoch nur eine untergeordnete Rolle, so dass vorliegend keine Unterscheidung des Erythrovirus in seine Genotypen erfolgen soll.

Die identifizierten microRNAs, deren Expression bei solchen Erythrovirus-induzierten Kardiomyopathien modifiziert sind, sind in der nachfolgenden Tabelle 9 aufgelistet. Hinsichtlich der Punktwerte und Signifikanzwerte wird auf die Erläuterungen zu den Beispielen 1 und 2 verwiesen.

**Tabelle 9: Expressionsmodifikationen von microRNAs bei einer Erythrovirus-induzierten Kardiomyopathie.**

| **SEQ ID NO** | **microRNA** | **Regulation ↑↓** | **Signifikanzwert** | **Punktwert** |
|---|---|---|---|---|
| 117 | hsa-miR-103b | hoch | 3 | 1 |
| 118 | hsa-miR-125a-5p | herunter | 3 | 1 |
| 119 | hsa-miR-1271-5p | herunter | 3 | 1 |
| 120 | hsa-miR-1274B | herunter | 6 | 4 |
| 121 | hsa-miR-138-1-3p | hoch | 3 | 1 |
| 122 | hsa-miR-146b-3p | hoch | 5 | 3 |
| 123 | hsa-miR-190a-5p | hoch | 4 | 1 |
| 124 | hsa-miR-199a-5p | hoch | 5 | 3 |
| 125 | hsa-miR-26b-3p | herunter | 3 | 1 |
| 126 | hsa-miR-320c | hoch | 5 | 3 |
| 127 | hsa-miR-3617-5p | hoch | 3 | 1 |
| 128 | hsa-miR-362-5p | hoch | 5 | 3 |
| 129 | hsa-miR-3663-3p | herunter | 3 | 1 |
| 130 | hsa-miR-367-3p | herunter | 3 | 1 |
| 131 | hsa-miR-3922-5p | hoch | 5 | 2 |
| 132 | hsa-miR-424-5p | hoch | 3 | 1 |
| 133 | hsa-miR-432-5p | herunter | 3 | 1 |
| 134 | hsa-miR-4429 | hoch | 5 | 3 |
| 135 | hsa-miR-4698 | hoch | 3 | 1 |
| 136 | hsa-miR-4726-3p | hoch | 3 | 1 |
| 137 | hsa-miR-4743-5p | hoch | 3 | 1 |
| 138 | hsa-miR-497-5p | hoch | 3 | 1 |
| 139 | hsa-miR-502-3p | herunter | 3 | 1 |
| 140 | hsa-miR-520d-3p | herunter | 3 | 1 |
| 141 | hsa-miR-520f-3p | hoch | 5 | 4 |
| 142 | hsa-miR-597-5p | hoch | 5 | 3 |
| 143 | hsa-miR-625-3p | herunter | 3 | 1 |
| 144 | hsa-miR-628-5p | hoch | 5 | 3 |
| 145 | hsa-miR-629-5p | hoch | 3 | 1 |
| 146 | hsa-miR-758-3p | hoch | 3 | 1 |
| 147 | mmu-miR-374-5p | hoch | 6 | 3 |

In einer Variante bezieht sich die Erfindung auf die Verwendung der in der Tabelle 9 mit einem Punktwert von 1 und/oder 2 und/oder 2 und/oder einem Signifikanzwert von 1 und/oder 2versehenen microRNAs einzeln oder in beliebiger Kombination miteinander als Marker zur Identifizierung einer Erythrovirus-induzierten Kardiomyopathie.

### Beispiel 7: Diagnose einer HHV6-induzierten Kardiomyopathie mittels einer Blutserum-/Blutplasmaprobe

Es wurde analog zu Beispiel 1 vorgegangen, nur dass die Blutzellenproben Patienten entnommen wurden, die an einer HHV6-induzierten Kardiomyopathie erkrankt waren. Hierin eingeschlossen sind Patienten mit einer ciHHV6-induzierten Kardiomyopathie.

ciHHV6 bezeichnet eine Sonderform des humanen Herpesvirus 6. Dieses Virus hat die Eigenschaft, sich in die Chromosomen des Wirts einzulagern; man spricht hier von chromosomaler Integration des HHV6 (ciHHV6).

Die identifizierten microRNAs, deren Expression bei solchen ciHHV6-induzierten Kardiomyopathien modifiziert sind, sind in der nachfolgenden Tabelle 10 aufgelistet.

In Tabelle 11 sind jene identifizierten microRNAs aufgelistet, welche bei einer myokardialen HHV6-Infektion verändert sind. Hinsichtlich der Punktwerte und Signifikanzwerte wird auf die Erläuterungen zu den Beispielen 1 und 2 verwiesen.

**Tabelle 10: Expressionsmodifikationen von microRNAs bei einer ciHHV6-induzierten Kardiomyopathie in einer Blutserum-/Blutplasmaprobe.**

| **SEQ ID NO** | **microRNA** | **Regulation ↑↓** | **Signifikanzwert** | **Punktwert** |
|---|---|---|---|---|
| 148 | hsa-let-7e-5p | hoch | 4 | 2 |
| 149 | hsa-miR-139-3p | hoch | 5 | 3 |
| 150 | hsa-miR-141-3p | hoch | 3 | 1 |
| 151 | hsa-miR-146b-3p | hoch | 5 | 3 |
| 152 | hsa-miR-181c-5p | hoch | 3 | 1 |
| 153 | hsa-miR-3156-5p | herunter | 3 | 1 |
| 154 | hsa-miR-362-5p | hoch | 5 | 3 |
| 155 | hsa-miR-411-5p | herunter | 4 | 3 |
| 156 | hsa-mir-422a | hoch | 4 | 4 |
| 157 | hsa-miR-4535 | herunter | 4 | 3 |
| 158 | hsa-miR-485-3p | hoch | 5 | 3 |
| 159 | hsa-miR-486-3p | hoch | 5 | 3 |
| 160 | hsa-miR-487b-3p | hoch | 5 | 3 |
| 161 | hsa-miR-494-3p | hoch | 5 | 3 |
| 162 | hsa-miR-511-5p | herunter | 3 | 1 |
| 163 | hsa-miR-520f-3p | herunter | 5 | 4 |
| 164 | hsa-miR-548c-3p | hoch | 5 | 3 |
| 165 | hsa-miR-548c-5p | hoch | 6 | 3 |
| 166 | hsa-miR-590-5p | herunter | 5 | 3 |
| 167 | hsa-miR-671-3p | hoch | 5 | 3 |
| 168 | hsa-miR-886-3p | hoch | 5 | 3 |
| 169 | hsa-miR-889-3p | hoch | 5 | 3 |
| 170 | hsa-miR-92a-3p | herunter | 6 | 3 |

**Tabelle 11: Expressionsmodifikationen von microRNAs bei einer HHV6-induzierten Kardiomyopathie in einer Blutserum-/Blutplasmaprobe.**

| **SEQ ID NO** | **microRNA** | **Regulation ↑↓** | **Signifikanzwert** | **Punktwert** |
|---|---|---|---|---|
| 171 | hsa-miR-192-5p | herunter | 3 | 1 |
| 172 | hsa-miR-29a-3p | herunter | 3 | 1 |
| 173 | hsa-miR-30c-5p | herunter | 3 | 1 |
| 174 | hsa-miR-483-5p | herunter | 5 | 3 |
| 175 | mmu-miR-374-5p | hoch | 6 | 3 |

In einer Variante bezieht sich die Erfindung auf die Verwendung der in den Tabellen 10 und 11 mit einem Punktwert von 1 und/oder 2 und/oder 2 und/oder einem Signifikanzwert von 1 und/oder 2 versehenen microRNAs einzeln oder in beliebiger Kombination miteinander als Marker zur Identifizierung einer HHV6-induzierten Kardiomyopathie, insbesondere einer Kardiomyopathie mit ciHHV6-Ausprägung.

In einer Variante bezieht sich die Erfindung auf die Verwendung der in der mit einem Punktwert von 1 und/oder 2 und/oder 2 und/oder einem Signifikanzwert von 1 und/oder 2 versehenen microRNAs einzeln oder in beliebiger Kombination miteinander.

## Patentansprüche

1. In-vitro-Verfahren zur Unterscheidung zwischen Patienten, bei denen zur diagnostischen Identifikation einer Herzmuskelerkrankung eine Myokardbiopsie erforderlich ist, und Patienten, bei denen keine Myokardbiopsie erforderlich ist, sowie zur optionalen differentialdiagnostischen Unterscheidung einzelner nicht-ischämischer Kardiomyopathien oder Speichererkrankungen des Herzens,
**dadurch gekennzeichnet,**
**dass** eine Blutserum-/Blutplasmaprobe eines Patienten unter Verwendung eines diagnostischen Profils analysiert wird, welches mindestens 2 microRNAs oder synthetische Nukleinsäuren mit identischen oder komplementären Sequenzen umfasst, die aus der Gruppe bestehend aus den folgenden microRNAs ausgewählt sind: hsa-let-7f-5p (SEQ ID NO: 10), hsa-miR-103a-3p (SEQ ID NO: 11), hsa-miR-197-3p (SEQ ID NO: 12), hsa-miR-215-5p (SEQ ID NO: 13), hsa-miR-223-3p (SEQ ID NO: 14), hsa-miR-23a-3p (SEQ ID NO: 15), hsa-miR-330-3p (SEQ ID NO: 16), hsa-miR-337-5p (SEQ ID NO: 17), hsa-miR-339-3p (SEQ ID NO: 18), hsa-miR-339-5p (SEQ ID NO: 19), hsa-miR-411-5p (SEQ ID NO: 20), hsa-miR-454-3p (SEQ ID NO: 21), hsa-miR-485-3p (SEQ ID NO: 22), hsa-miR-487b-3p (SEQ ID NO: 23), hsa-miR-494-3p (SEQ ID NO: 24), hsa-miR-505-3p (SEQ ID NO: 25), hsa-miR-628-5p (SEQ ID NO: 26), hsa-miR-671-3p (SEQ ID NO: 27), hsa-miR-744-5p (SEQ ID NO: 28), hsa-miR-889-3p (SEQ ID NO: 29), mmu-miR-495-3p (SEQ ID NO: 30).

2. In-vitro-Verfahren gemäß Anspruch 1, **dadurch gekennzeichnet, dass** die nicht-ischämischen Kardiomyopathien oder Speichererkrankungen des Herzens ausgewählt sind aus der Gruppe umfassend Adenovirus-induzierte Kardiomyopathie, Enterovirus-(Coxsackievirus-)induzierte Kardiomyopathie, ciHHv6/HHV6-Virus-induzierte Kardiomyopathie, Erythrovirus-induzierte Kardiomyopathie, Myokarditis mit Aufkommen von myokardialen Riesenzellen, kardialer Sarkoidose, Amyloidose des Herzens.

3. In-vitro-Verfahren gemäß Anspruch 1 oder 2, **dadurch gekennzeichnet, dass** das diagnostische Profil zur Identifikation einer Adenovirus-induzierten Kardiomyopathie zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-miR-1274B (SEQ ID NO: 31), hsa-miR-136-3p (SEQ ID NO: 32), hsa-miR-187-3p (SEQ ID NO: 33), hsa-miR-199a-5p (SEQ ID NO: 34), hsa-miR-215-5p (SEQ ID NO: 35), hsa-mir-422a (SEQ ID NO: 36), hsa-miR-4449 (SEQ ID NO: 37), hsa-miR-4674 (SEQ ID NO: 38), hsa-miR-5096 (SEQ ID NO: 39), hsa-miR-548c-3p (SEQ ID NO: 40), hsa-miR-548c-5p (SEQ ID NO: 41), hsa-miR-650 (SEQ ID NO: 42).

4. In-vitro-Verfahren gemäß einem der vorherigen Ansprüche, **dadurch gekennzeichnet, dass** das diagnostische Profil zur Identifikation einer Enterovirus-(Coxsackievirus-)induzierten Kardiomyopathie zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-miR-1180-3p (SEQ ID NO: 43), hsa-miR-1-3p (SEQ ID NO: 44), hsa-miR-140-5p (SEQ ID NO: 45), hsa-miR-150-3p (SEQ ID NO: 46), hsa-miR-16-5p (SEQ ID NO: 47), hsa-miR-215-5p (SEQ ID NO: 48), hsa-miR-25-3p (SEQ ID NO: 49), hsa-miR-30a-5p (SEQ ID NO: 50), hsa-miR-30e-5p (SEQ ID NO: 51), hsa-miR-3138 (SEQ ID NO: 52), hsa-miR-3622b-5p (SEQ ID NO: 53), hsa-miR-3922-5p (SEQ ID NO: 54), hsa-mir-422a (SEQ ID NO: 55), hsa-miR-4274 (SEQ ID NO: 56), hsa-miR-4303 (SEQ ID NO: 57), hsa-miR-4419b (SEQ ID NO: 58), hsa-miR-4429 (SEQ ID NO: 59), hsa-miR-4467 (SEQ ID NO: 60), hsa-miR-4651 (SEQ ID NO: 61), hsa-miR-4716-5p (SEQ ID NO: 62), hsa-miR-548b-5p (SEQ ID NO: 63), hsa-miR-548c-3p (SEQ ID NO: 64), hsa-miR-597-5p (SEQ ID NO: 65), hsa-miR-601 (SEQ ID NO: 66), hsa-miR-629-3p (SEQ ID NO: 67), hsa-miR-660-5p (SEQ ID NO: 68), hsa-miR-886-3p (SEQ ID NO: 69), hsa-miR-923 (SEQ ID NO: 70), mmu-miR-374-5p (SEQ ID NO: 71).

5. In-vitro-Verfahren gemäß einem der vorherigen Ansprüche, **dadurch gekennzeichnet, dass** das diagnostische Profil zur Identifikation einer Amyloidose des Herzens zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-miR-103a-3p (SEQ ID NO: 72), hsa-miR-106b-5p (SEQ ID NO: 73), hsa-miR-126-3p (SEQ ID NO: 74), hsa-miR-126-5p (SEQ ID NO: 75), hsa-miR-1 274A (SEQ ID NO: 76), hsa-miR-1274B (SEQ ID NO: 77), hsa-miR-132-3p (SEQ ID NO: 78), hsa-miR-140-3p (SEQ ID NO: 79), hsa-miR-142-3p (SEQ ID NO: 80), hsa-miR-146b-5p (SEQ ID NO: 81), hsa-miR-150-5p (SEQ ID NO: 82), hsa-miR-15b-5p (SEQ ID NO: 83), hsa-miR-181b-5p (SEQ ID NO: 84), hsa-miR-186-5p (SEQ ID NO: 85), hsa-miR-21-5p (SEQ ID NO: 86), hsa-miR-222-3p (SEQ ID NO: 87), hsa-miR-223-5p (SEQ ID NO: 88), hsa-miR-24-3p (SEQ ID NO: 89), hsa-miR-26b-5p (SEQ ID NO: 90), hsa-miR-30a-3p (SEQ ID NO: 91), hsa-miR-30d-5p (SEQ ID NO: 92), hsa-miR-320a (SEQ ID NO: 93), hsa-miR-320c (SEQ ID NO: 94), hsa-miR-328-3p (SEQ ID NO: 95), hsa-miR-375 (SEQ ID NO: 96), hsa-miR-378-5p (SEQ ID NO: 97), hsa-mir-423 (SEQ ID NO: 98), hsa-miR-4286 (SEQ ID NO: 99), hsa-miR-451 a (SEQ ID NO: 100), hsa-miR-4535 (SEQ ID NO: 101), hsa-miR-483-5p (SEQ ID NO: 102), hsa-miR-518d-3p (SEQ ID NO: 103), hsa-miR-532-5p (SEQ ID NO: 104), hsa-miR-590-5p (SEQ ID NO: 105), hsa-miR-601 (SEQ ID NO: 106), hsa-miR-660-5p (SEQ ID NO: 107), hsa-miR-885-5p (SEQ ID NO: 108), hsa-miR-92a-3p (SEQ ID NO: 109), hsa-miR-99b-5p (SEQ ID NO: 110).

6. In-vitro-Verfahren gemäß einem der vorherigen Ansprüche, **dadurch gekennzeichnet, dass** das diagnostische Profil zur Identifikation einer Lambda-Amyloidose des Herzens zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-miR-106b-5p (SEQ ID NO: 73), hsa-miR-1274A (SEQ ID NO: 76), hsa-miR-30a-3p (SEQ ID NO: 91), hsa-miR-4535 (SEQ ID NO: 101).

7. In-vitro-Verfahren gemäß einem der vorherigen Ansprüche, **dadurch gekennzeichnet, dass** das diagnostische Profil zur Identifikation einer ATTR-Amyloidose des Herzens zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-miR-140-3p (SEQ ID NO: 79), hsa-miR-181b-5p (SEQ ID NO: 84), hsa-miR-222-3p (SEQ ID NO: 87), hsa-miR-223-5p (SEQ ID NO: 88).

8. In-vitro-Verfahren gemäß einem der vorherigen Ansprüche, **dadurch gekennzeichnet, dass** das diagnostische Profil zur Identifikation einer kardialen Sarkoidose/Riesenzellenmyokarditis des Herzens zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-miR-139-3p (SEQ ID NO: 111), hsa-miR-210-3p (SEQ ID NO: 112), hsa-miR-296-5p (SEQ ID NO: 113), hsa-miR-486-3p (SEQ ID NO: 114), hsa-miR-500a-5p (SEQ ID NO: 115), hsa-miR-542-3p (SEQ ID NO: 116).

9. In-vitro-Verfahren gemäß einem der vorherigen Ansprüche, **dadurch gekennzeichnet, dass** das diagnostische Profil zur Identifikation einer Erythrovirus-induzierten Kardiomyopathie zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-miR-103b (SEQ ID NO: 117), hsa-miR-125a-5p (SEQ ID NO: 118), hsa-miR-1271-5p (SEQ ID NO: 119), hsa-miR-1274B (SEQ ID NO: 120), hsa-miR-138-1-3p (SEQ ID NO: 121), hsa-miR-146b-3p (SEQ ID NO: 122), hsa-miR-190a-5p (SEQ ID NO: 123), hsa-miR-199a-5p (SEQ ID NO: 124), hsa-miR-26b-3p (SEQ ID NO: 125), hsa-miR-320c (SEQ ID NO: 126), hsa-miR-3617-5p (SEQ ID NO: 127), hsa-miR-362-5p (SEQ ID NO: 128), hsa-miR-3663-3p (SEQ ID NO: 129), hsa-miR-367-3p (SEQ ID NO: 130), hsa-miR-3922-5p (SEQ ID NO: 131), hsa-miR-424-5p (SEQ ID NO: 132), hsa-miR-432-5p (SEQ ID NO: 133), hsa-miR-4429 (SEQ ID NO: 134), hsa-miR-4698 (SEQ ID NO: 135), hsa-miR-4726-3p (SEQ ID NO: 136), hsa-miR-4743-5p (SEQ ID NO: 137), hsa-miR-497-5p (SEQ ID NO: 138), hsa-miR-502-3p (SEQ ID NO: 139), hsa-miR-520d-3p (SEQ ID NO: 140), hsa-miR-520f-3p (SEQ ID NO: 141), hsa-miR-597-5p (SEQ ID NO: 142), hsa-miR-625-3p (SEQ ID NO: 143), hsa-miR-628-5p (SEQ ID NO: 144), hsa-miR-629-5p (SEQ ID NO: 145), hsa-miR-758-3p (SEQ ID NO: 146), mmu-miR-374-5p (SEQ ID NO: 147).

10. In-vitro-Verfahren gemäß einem der vorherigen Ansprüche, **dadurch gekennzeichnet, dass** das diagnostische Profil zur Identifikation einer ciHHV6-induzierten Kardiomyopathie zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-let-7e-5p (SEQ ID NO: 148), hsa-miR-139-3p (SEQ ID NO: 149), hsa-miR-141-3p (SEQ ID NO: 150), hsa-miR-146b-3p (SEQ ID NO: 151), hsa-miR-181c-5p (SEQ ID NO: 152), hsa-miR-3156-5p (SEQ ID NO: 153), hsa-miR-362-5p (SEQ ID NO: 154), hsa-miR-411-5p (SEQ ID NO: 155), hsa-mir-422a (SEQ ID NO: 156), hsa-miR-4535 (SEQ ID NO: 157), hsa-miR-485-3p (SEQ ID NO: 158), hsa-miR-486-3p (SEQ ID NO: 159), hsa-miR-487b-3p (SEQ ID NO: 160), hsa-miR-494-3p (SEQ ID NO: 161), hsa-miR-511-5p (SEQ ID NO: 162), hsa-miR-520f-3p (SEQ ID NO: 163), hsa-miR-548c-3p (SEQ ID NO: 164), hsa-miR-548c-5p (SEQ ID NO: 165), hsa-miR-590-5p (SEQ ID NO: 166), hsa-miR-671-3p (SEQ ID NO: 167), hsa-miR-886-3p (SEQ ID NO: 168), hsa-miR-889-3p (SEQ ID NO: 169), hsa-miR-92a-3p (SEQ ID NO: 170).

11. In-vitro-Verfahren gemäß einem der vorherigen Ansprüche, **dadurch gekennzeichnet, dass** das diagnostische Profil zur Identifikation einer HHV6-induzierten Kardiomyopathie zusätzlich mindestens 3 microRNAs oder synthetische Nukleinsäuren mit identischen oder komplementären Sequenzen umfasst, die aus der Gruppe bestehend aus den folgenden microRNAs ausgewählt sind: hsa-miR-192-5p (SEQ ID NO: 171), hsa-miR-29a-3p (SEQ ID NO: 172), hsa-miR-30c-5p (SEQ ID NO: 173), hsa-miR-483-5p (SEQ ID NO: 174), mmu-miR-374-5p (SEQ ID NO: 175).

12. In-vitro-Verfahren gemäß einem der vorherigen Ansprüche, **dadurch gekennzeichnet, dass** die folgenden Schritte durchgeführt werden:
- Bereitstellung einer Probe, die von einem Patienten, bei dem ein Verdacht auf das Vorliegen einer nicht-ischämischen Kardiomyopathie oder einer Speichererkrankung des Herzens vorliegt, gewonnen wurde,
- Inkontaktbringen der Probe mit mindestens zwei Sonden, die jeweils eine Sequenz aufweisen, welche den Sequenzen der microRNAs aus zumindest einer der Gruppen gemäß einem der Ansprüche 1 bis 11 entspricht oder zu diesen komplementär ist, unter Bedingungen, die eine Hybridisierung zwischen in der Probe enthaltenen microRNAs und den Sonden erlauben,
- Ermittlung einer erfolgten Hybridisierung zwischen microRNAs der Probe und der mindestens einen Sonde und
- Bestimmung des Vorhandenseins einer microRNA in der Probe anhand des Hybridisierungsergebnisses des vorherigen Schritts.

## Claims

1. An in-vitro method for differentiating between patients requiring, for the diagnostic identification of a myocardial disorder, myocardial biopsy, and patients not requiring myocardial biopsy, and for the optional differential diagnostic differentiation between individual non-ischemic cardiomyopathies or storage diseases of the heart,
**characterized in**
**that** a blood serum/blood plasma sample of a patient is analyzed using a diagnostic profile comprising at least 2 microRNAs or synthetic nucleic acids having identical or complementary sequences, selected from the group consisting of the following microRNAs: hsa-let-7f-5p (SEQ ID NO: 10), hsa-miR-103a-3p (SEQ ID NO: 11), hsa-miR-197-3p (SEQ ID NO: 12), hsa-miR-215-5p (SEQ ID NO: 13), hsa-miR-223-3p (SEQ ID NO: 14), hsa-miR-23a-3p (SEQ ID NO: 15), hsa-miR-330-3p (SEQ ID NO: 16), hsa-miR-337-5p (SEQ ID NO: 17), hsa-miR-339-3p (SEQ ID NO: 18), hsa-miR-339-5p (SEQ ID NO: 19), hsa-miR-411-5p (SEQ ID NO: 20), hsa-miR-454-3p (SEQ ID NO: 21), hsa-miR-485-3p (SEQ ID NO: 22), hsa-miR-487b-3p (SEQ ID NO: 23), hsa-miR-494-3p (SEQ ID NO: 24), hsa-miR-505-3p (SEQ ID NO: 25), hsa-miR-628-5p (SEQ ID NO: 26), hsa-miR-671-3p (SEQ ID NO: 27), hsa-miR-744-5p (SEQ ID NO: 28), hsa-miR-889-3p (SEQ ID NO: 29), mmu-miR-495-3p (SEQ ID NO: 30).

2. The in-vitro method as claimed in claim 1, **characterized in that** the non-ischemic cardiomyopathies or storage diseases of the heart are selected from the group comprising adenovirus-induced cardiomyopathy, enterovirus-(coxsackievirus-)induced cardiomyopathy, ciHHv6/HHV6-virus-induced cardiomyopathy, erythrovirus-induced cardiomyopathy, myocarditis with presence of myocardial giant cells, cardiac sarcoidosis, amyloidosis of the heart.

3. The in-vitro method as claimed in claim 1 or 2, **characterized in that** the diagnostic profile for identifying an adenovirus-induced cardiomyopathy additionally comprises at least 3 microRNAs or synthetic nucleic acids having identical or complementary sequences, selected from the group consisting of the following microRNAs: hsa-miR-1274B (SEQ ID NO: 31), hsa-miR-136-3p (SEQ ID NO: 32), hsa-miR-187-3p (SEQ ID NO: 33), hsa-miR-199a-5p (SEQ ID NO: 34), hsa-miR-215-5p (SEQ ID NO: 35), hsa-miR-422a (SEQ ID NO: 36), hsa-miR-4449 (SEQ ID NO: 37), hsa-miR-4674 (SEQ ID NO: 38), hsa-miR-5096 (SEQ ID NO: 39), hsa-miR-548c-3p (SEQ ID NO: 40), hsa-miR-548c-5p (SEQ ID NO: 41), hsa-miR-650 (SEQ ID NO: 42).

4. The in-vitro method as claimed in any of the preceding claims, **characterized in that** the diagnostic profile for identifying an enterovirus-(coxsackievirus-)induced cardiomyopathy additionally comprises at least 3 microRNAs or synthetic nucleic acids having identical or complementary sequences, selected from the group consisting of the following microRNAs: hsa-miR-1180-3p (SEQ ID NO: 43), hsa-miR-1-3p (SEQ ID NO: 44), hsa-miR-140-5p (SEQ ID NO: 45), hsa-miR-150-3p (SEQ ID NO: 46), hsa-miR-16-5p (SEQ ID NO: 47), hsa-miR-215-5p (SEQ ID NO: 48), hsa-miR-25-3p (SEQ ID NO: 49), hsa-miR-30a-5p (SEQ ID NO: 50), hsa-miR-30e-5p (SEQ ID NO: 51), hsa-miR-3138 (SEQ ID NO: 52), hsa-miR-3622b-5p (SEQ ID NO: 53), hsa-miR-3922-5p (SEQ ID NO: 54), hsa-miR-422a (SEQ ID NO: 55), hsa-miR-4274 (SEQ ID NO: 56), hsa-miR-4303 (SEQ ID NO: 57), hsa-miR-4419b (SEQ ID NO: 58), hsa-miR-4429 (SEQ ID NO: 59), hsa-miR-4467 (SEQ ID NO: 60), hsa-miR-4651 (SEQ ID NO: 61), hsa-miR-4716-5p (SEQ ID NO: 62), hsa-miR-548b-5p (SEQ ID NO: 63), hsa-miR-548c-3p (SEQ ID NO: 64), hsa-miR-597-5p (SEQ ID NO: 65), hsa-miR-601 (SEQ ID NO: 66), hsa-miR-629-3p (SEQ ID NO: 67), hsa-miR-660-5p (SEQ ID NO: 68), hsa-miR-886-3p (SEQ ID NO: 69), hsa-miR-923 (SEQ ID NO: 70), mmu-miR-374-5p (SEQ ID NO: 71).

5. The in-vitro method as claimed in any of the preceding claims, **characterized in that** the diagnostic profile for identifying amyloidosis of the heart additionally comprises at least 3 microRNAs or synthetic nucleic acids having identical or complementary sequences, selected from the group consisting of the following microRNAs: hsa-miR-103a-3p (SEQ ID NO: 72), hsa-miR-106b-5p (SEQ ID NO: 73), hsa-miR-126-3p (SEQ ID NO: 74), hsa-miR-126-5p (SEQ ID NO: 75), hsa-miR-1274A (SEQ ID NO: 76), hsa-miR-1274B (SEQ ID NO: 77), hsa-miR-132-3p (SEQ ID NO: 78), hsa-miR-140-3p (SEQ ID NO: 79), hsa-miR-142-3p (SEQ ID NO: 80), hsa-miR-146b-5p (SEQ ID NO: 81), hsa-miR-150-5p (SEQ ID NO: 82), hsa-miR-15b-5p (SEQ ID NO: 83), hsa-miR-181 b-5p (SEQ ID NO: 84), hsa-miR-186-5p (SEQ ID NO: 85), hsa-miR-21-5p (SEQ ID NO: 86), hsa-miR-222-3p (SEQ ID NO: 87), hsa-miR-223-5p (SEQ ID NO: 88), hsa-miR-24-3p (SEQ ID NO: 89), hsa-miR-26b-5p (SEQ ID NO: 90), hsa-miR-30a-3p (SEQ ID NO: 91), hsa-miR-30d-5p (SEQ ID NO: 92), hsa-miR-320a (SEQ ID NO: 93), hsa-miR-320c (SEQ ID NO: 94), hsa-miR-328-3p (SEQ ID NO: 95), hsa-miR-375 (SEQ ID NO: 96), hsa-miR-378-5p (SEQ ID NO: 97), hsa-mir-423 (SEQ ID NO: 98), hsa-miR-4286 (SEQ ID NO: 99), hsa-miR-451a (SEQ ID NO: 100), hsa-miR-4535 (SEQ ID NO: 101), hsa-miR-483-5p (SEQ ID NO: 102), hsa-miR-518d-3p (SEQ ID NO: 103), hsa-miR-532-5p (SEQ ID NO: 104), hsa-miR-590-5p (SEQ ID NO: 105), hsa-miR-601 (SEQ ID NO: 106), hsa-miR-660-5p (SEQ ID NO: 107), hsa-miR-885-5p (SEQ ID NO: 108), hsa-miR-92a-3p (SEQ ID NO: 109), hsa-miR-99b-5p (SEQ ID NO: 110).

6. The in-vitro method as claimed in any of the preceding claims, **characterized in that** the diagnostic profile for identifying lambda amyloidosis of the heart additionally comprises at least 3 microRNAs or synthetic nucleic acids having identical or complementary sequences, selected from the group consisting of the following microRNAs: hsa-miR-106b-5p (SEQ ID NO: 73), hsa-miR-1274A (SEQ ID NO: 76), hsa-miR-30a-3p (SEQ ID NO: 91), hsa-miR-4535 (SEQ ID NO: 101).

7. The in-vitro method as claimed in any of the preceding claims, **characterized in that** the diagnostic profile for identifying ATTR amyloidosis of the heart additionally comprises at least 3 microRNAs or synthetic nucleic acids having identical or complementary sequences, selected from the group consisting of the following microRNAs: hsa-miR-140-3p (SEQ ID NO: 79), hsa-miR-181b-5p (SEQ ID NO: 84), hsa-miR-222-3p (SEQ ID NO: 87), hsa-miR-223-5p (SEQ ID NO: 88).

8. The in-vitro method as claimed in any of the preceding claims, **characterized in that** the diagnostic profile for identifying cardiac sarcoidosis/giant cell myocarditis of the heart additionally comprises at least 3 microRNAs or synthetic nucleic acids having identical or complementary sequences, selected from the group consisting of the following microRNAs: hsa-miR-139-3p (SEQ ID NO: 111), hsa-miR-210-3p (SEQ ID NO: 112), hsa-miR-296-5p (SEQ ID NO: 113), hsa-miR-486-3p (SEQ ID NO: 114), hsa-miR-500a-5p (SEQ ID NO: 115), hsa-miR-542-3p (SEQ ID NO: 116).

9. The in-vitro method as claimed in any of the preceding claims, **characterized in that** the diagnostic profile for identifying erythrovirus-induced cardiomyopathy additionally comprises at least 3 microRNAs or synthetic nucleic acids having identical or complementary sequences, selected from the group consisting of the following microRNAs: hsa-miR-103b (SEQ ID NO: 117), hsa-miR-125a-5p (SEQ ID NO: 118), hsa-miR-1271-5p (SEQ ID NO: 119), hsa-miR-1274B (SEQ ID NO: 120), hsa-miR-138-1-3p (SEQ ID NO: 121), hsa-miR-146b-3p (SEQ ID NO: 122), hsa-miR-190a-5p (SEQ ID NO: 123), hsa-miR-199a-5p (SEQ ID NO: 124), hsa-miR-26b-3p (SEQ ID NO: 125), hsa-miR-320c (SEQ ID NO: 126), hsa-miR-3617-5p (SEQ ID NO: 127), hsa-miR-362-5p (SEQ ID NO: 128), hsa-miR-3663-3p (SEQ ID NO: 129), hsa-miR-367-3p (SEQ ID NO: 130), hsa-miR-3922-5p (SEQ ID NO: 131), hsa-miR-424-5p (SEQ ID NO: 132), hsa-miR-432-5p (SEQ ID NO: 133), hsa-miR-4429 (SEQ ID NO: 134), hsa-miR-4698 (SEQ ID NO: 135), hsa-miR-4726-3p (SEQ ID NO: 136), hsa-miR-4743-5p (SEQ ID NO: 137), hsa-miR-497-5p (SEQ ID NO: 138), hsa-miR-502-3p (SEQ ID NO: 139), hsa-miR-520d-3p (SEQ ID NO: 140), hsa-miR-520f-3p (SEQ ID NO: 141), hsa-miR-597-5p (SEQ ID NO: 142), hsa-miR-625-3p (SEQ ID NO: 143), hsa-miR-628-5p (SEQ ID NO: 144), hsa-miR-629-5p (SEQ ID NO: 145), hsa-miR-758-3p (SEQ ID NO: 146), mmu-miR-374-5p (SEQ ID NO: 147).

10. The in-vitro method as claimed in any of the preceding claims, **characterized in that** the diagnostic profile for identifying ciHHV6-induced cardiomyopathy additionally comprises at least 3 microRNAs or synthetic nucleic acids having identical or complementary sequences, selected from the group consisting of the following microRNAs: hsa-let-7e-5p (SEQ ID NO: 148), hsa-miR-139-3p (SEQ ID NO: 149), hsa-miR-141-3p (SEQ ID NO: 150), hsa-miR-146b-3p (SEQ ID NO: 151), hsa-miR-181c-5p (SEQ ID NO: 152), hsa-miR-3156-5p (SEQ ID NO: 153), hsa-miR-362-5p (SEQ ID NO: 154), hsa-miR-411-5p (SEQ ID NO: 155), hsa-miR-422a (SEQ ID NO: 156), hsa-miR-4535 (SEQ ID NO: 157), hsa-miR-485-3p (SEQ ID NO: 158), hsa-miR-486-3p (SEQ ID NO: 159), hsa-miR-487b-3p (SEQ ID NO: 160), hsa-miR-494-3p (SEQ ID NO: 161), hsa-miR-511-5p (SEQ ID NO: 162), hsa-miR-520f-3p (SEQ ID NO: 163), hsa-miR-548c-3p (SEQ ID NO: 164), hsa-miR-548c-5p (SEQ ID NO: 165), hsa-miR-590-5p (SEQ ID NO: 166), hsa-miR-671-3p (SEQ ID NO: 167), hsa-miR-886-3p (SEQ ID NO: 168), hsa-miR-889-3p (SEQ ID NO: 169), hsa-miR-92a-3p (SEQ ID NO: 170).

11. The in-vitro method as claimed in any of the preceding claims, **characterized in that** the diagnostic profile for identifying HHV6-induced cardiomyopathy additionally comprises at least 3 microRNAs or synthetic nucleic acids having identical or complementary sequences, selected from the group consisting of the following microRNAs: hsa-miR-192-5p (SEQ ID NO: 171), hsa-miR-29a-3p (SEQ ID NO: 172), hsa-miR-30c-5p (SEQ ID NO: 173), hsa-miR-483-5p (SEQ ID NO: 174), mmu-miR-374-5p (SEQ ID NO: 175).

12. The in-vitro method as claimed in any of the preceding claims, **characterized in that** the following steps are carried out:
- providing a sample obtained from a patient suspected of suffering from non-ischemic cardiomyopathy or a storage disease of the heart,
- bringing the sample into contact with at least two probes each comprising a sequence corresponding to the sequences of the microRNAs from at least one of the groups as set forth in any of claims 1 to 111 or complementary to these, under conditions allowing hybridization between microRNAs contained in the sample and the probes,
- determining hybridization between microRNAs of the sample and the at least one probe and
- confirming the presence of a microRNA in the sample using the hybridization result of the previous step.

## Revendications

1. Procédé in vitro pour faire la distinction entre des patients nécessitant une biopsie du myocarde pour l'identification diagnostique d'une maladie du myocarde et des patients ne nécessitant pas une biopsie du myocarde, ainsi que pour la différenciation facultative par diagnostic différentiel de formes individuelles de cardiomyopathies non ischémiques ou de maladies de surcharge du cœur,
**caractérisé en ce que**
un échantillon de sérum sanguin/plasma sanguin d'un patient est analysé en utilisant un profil diagnostique qui comprend au moins 2 microARN ou acides nucléiques synthétiques ayant des séquences identiques ou complémentaires qui sont sélectionnées parmi le groupe composé des microARN suivants : hsa-let-7f-5p (SEQ ID N° : 10), hsa-miR-103a-3p (SEQ ID N°: 11), hsa-miR-197-3p (SEQ ID N°: 12), hsa-miR-215-5p (SEQ ID N°: 13), hsa-miR-223-3p (SEQ ID N°: 14), hsa-miR-23a-3p (SEQ ID N°: 15), hsa-miR-330-3p (SEQ ID N°: 16), hsa-miR-337-5p (SEQ ID N°: 17), hsa-miR-339-3p (SEQ ID N°: 18), hsa-miR-339-5p (SEQ ID N°: 19), hsa-miR-411-5p (SEQ ID N°: 20), hsa-miR-454-3p (SEQ ID N°: 21), hsa-miR-485-3p (SEQ ID N°: 22), hsa-miR-487b-3p (SEQ ID N°: 23), hsa-miR-494-3p (SEQ ID N°: 24), hsa-miR-505-3p (SEQ ID N° : 25), hsa-miR-628-5p (SEQ ID N° : 26), hsa-miR-671-3p (SEQ ID N° : 27), hsa-miR-744-5p (SEQ ID N° : 28), hsa-miR-889-3p (SEQ ID N° : 29), mmu-miR-495-3p (SEQ ID N° : 30).

2. Procédé in vitro selon la revendication 1, **caractérisé en ce que** les cardiomyopathies non ischémiques ou maladies de surcharge du cœur sont sélectionnées parmi le groupe comprenant la cardiomyopathie induite par adénovirus, la cardiomyopathie induite par entérovirus-(virus Coxsackie), la cardiomyopathie induite par virus ciHHv6/HHV6, la cardiomyopathie induite par érythrovirus, la myocardite avec apparition de cellules géantes du myocarde, la sarcoïdose cardiaque, l'amylose du cœur.

3. Procédé in vitro selon la revendication 1 ou 2, **caractérisé en ce que** le profil diagnostique pour l'identification d'une cardiomyopathie induite par adénovirus comprend en outre au moins 3 microARN ou acides nucléiques synthétiques ayant des séquences identiques ou complémentaires qui sont sélectionnées parmi le groupe composé des microARN suivants : hsa-miR-1274B (SEQ ID N°: 31), hsa-miR-136-3p (SEQ ID N°: 32), hsa-miR-187-3p (SEQ ID N° : 33), hsa-miR-199a-5p (SEQ ID N° : 34), hsa-miR-215-5p (SEQ ID N° : 35), hsa-mir-422a (SEQ ID N° : 36), hsa-miR-4449 (SEQ ID N° : 37), hsa-miR-4674 (SEQ ID N° : 38), hsa-miR-5096 (SEQ ID N° : 39), hsa-miR-548c-3p (SEQ ID N°: 40), hsa-miR-548c-5p (SEQ ID N°: 41), hsa-miR-650 (SEQ ID N°: 42).

4. Procédé in vitro selon l'une des revendications précédentes, **caractérisé en ce que** le profil diagnostique pour l'identification d'une cardiomyopathie induite par entérovirus-(virus Coxsackie) comprend en outre au moins 3 microARN ou acides nucléiques synthétiques ayant des séquences identiques ou complémentaires qui sont sélectionnées parmi le groupe composé des microARN suivants : hsa-miR-1180-3p (SEQ ID N°: 43), hsa-miR-1-3p (SEQ ID N° : 44), hsa-miR-140-5p (SEQ ID N° : 45), hsa-miR-150-3p (SEQ ID N°: 46), hsa-miR-16-5p (SEQ ID N°: 47), hsa-miR-215-5p (SEQ ID N°: 48), hsa-miR-25-3p (SEQ ID N°: 49), hsa-miR-30a-5p (SEQ ID N°: 50), hsa-miR-30e-5p (SEQ ID N°: 51), hsa-miR-3138 (SEQ ID N°: 52), hsa-miR-3622b-5p (SEQ ID N°: 53), hsa-miR-3922-5p (SEQ ID N°: 54), hsa-mir-422a (SEQ ID N°: 55), hsa-miR-4274 (SEQ ID N°: 56), hsa-miR-4303 (SEQ ID N°: 57), hsa-miR-4419b (SEQ ID N°: 58), hsa-miR-4429 (SEQ ID N°: 59), hsa-miR-4467 (SEQ ID N°: 60), hsa-miR-4651 (SEQ ID N°: 61), hsa-miR-4716-5p (SEQ ID N°: 62), hsa-miR-548b-5p (SEQ ID N°: 63), hsa-miR-548c-3p (SEQ ID N°: 64), hsa-miR-597-5p (SEQ ID N°: 65), hsa-miR-601 (SEQ ID N°: 66), hsa-miR-629-3p (SEQ ID N° : 67), hsa-miR-660-5p (SEQ ID N° : 68), hsa-miR-886-3p (SEQ ID N°: 69), hsa-miR-923 (SEQ ID N°: 70), mmu-miR-374-5p (SEQ ID N°: 71).

5. Procédé in vitro selon l'une des revendications précédentes, **caractérisé en ce que** le profil diagnostique pour l'identification d'une amylose du cœur comprend en outre au moins 3 microARN ou acides nucléiques synthétiques ayant des séquences identiques ou complémentaires qui sont sélectionnées parmi le groupe composé des microARN suivants : hsa-miR-103a-3p (SEQ ID N° : 72), hsa-miR-106b-5p (SEQ ID N° : 73), hsa-miR-126-3p (SEQ ID N°: 74), hsa-miR-126-5p (SEQ ID N°: 75), hsa-miR-1274A (SEQ ID N°: 76), hsa-miR-1274B (SEQ ID N°: 77), hsa-miR-132-3p (SEQ ID N°: 78), hsa-miR-140-3p (SEQ ID N° : 79), hsa-miR-142-3p (SEQ ID N° : 80), hsa-miR-146b-5p (SEQ ID N°: 81), hsa-miR-150-5p (SEQ ID N°: 82), hsa-miR-15b-5p (SEQ ID N°: 83), hsa-miR-181b-5p (SEQ ID N°: 84), hsa-miR-186-5p (SEQ ID N°: 85), hsa-miR-21-5p (SEQ ID N°: 86), hsa-miR-222-3p (SEQ ID N°: 87), hsa-miR-223-5p (SEQ ID N°: 88), hsa-miR-24-3p (SEQ ID N°: 89), hsa-miR-26b-5p (SEQ ID N°: 90), hsa-miR-30a-3p (SEQ ID N°: 91), hsa-miR-30d-5p (SEQ ID N° : 92), hsa-miR-320a (SEQ ID N° : 93), hsa-miR-320c (SEQ ID N° : 94), hsa-miR-328-3p (SEQ ID N°: 95), hsa-miR-375 (SEQ ID N°: 96), hsa-miR-378-5p (SEQ ID N°: 97), hsa-mir-423 (SEQ ID N°: 98), hsa-miR-4286 (SEQ ID N° : 99), hsa-miR-451a (SEQ ID N° : 100), hsa-miR-4535 (SEQ ID N° : 101), hsa-miR-483-5p (SEQ ID N° : 102), hsa-miR-518d-3p (SEQ ID N° : 103), hsa-miR-532-5p (SEQ ID N°: 104), hsa-miR-590-5p (SEQ ID N°: 105), hsa-miR-601 (SEQ ID N°: 106), hsa-miR-660-5p (SEQ ID N°: 107), hsa-miR-885-5p (SEQ ID N°: 108), hsa-miR-92a-3p (SEQ ID N°: 109), hsa-miR-99b-5p (SEQ ID N° : 110).

6. Procédé in vitro selon l'une des revendications précédentes, **caractérisé en ce que** le profil diagnostique pour l'identification d'une amylose lambda du cœur comprend en outre au moins 3 microARN ou acides nucléiques synthétiques ayant des séquences identiques ou complémentaires qui sont sélectionnées parmi le groupe composé des microARN suivants : hsa-miR-106b-5p (SEQ ID N°: 73), hsa-miR-1274A (SEQ ID N°: 76), hsa-miR-30a-3p (SEQ ID N° : 91), hsa-miR-4535 (SEQ ID N°: 101).

7. Procédé in vitro selon l'une des revendications précédentes, **caractérisé en ce que** le profil diagnostique pour l'identification d'une amylose ATTR du cœur comprend en outre au moins 3 microARN ou acides nucléiques synthétiques ayant des séquences identiques ou complémentaires qui sont sélectionnées parmi le groupe composé des microARN suivants : hsa-miR-140-3p (SEQ ID N°: 79), hsa-miR-181b-5p (SEQ ID N°: 84), hsa-miR-222-3p (SEQ ID N° : 87), hsa-miR-223-5p (SEQ ID N° : 88).

8. Procédé in vitro selon l'une des revendications précédentes, **caractérisé en ce que** le profil diagnostique pour l'identification d'une sarcoïdose cardiaque/myocardite à cellules géantes du cœur comprend en outre au moins 3 microARN ou acides nucléiques synthétiques ayant des séquences identiques ou complémentaires qui sont sélectionnées parmi le groupe composé des microARN suivants : hsa-miR-139-3p (SEQ ID N°: 111), hsa-miR-210-3p (SEQ ID N°: 112), hsa-miR-296-5p (SEQ ID N°: 113), hsa-miR-486-3p (SEQ ID N°: 114), hsa-miR-500a-5p (SEQ ID N°: 115), hsa-miR-542-3p (SEQ ID N°: 116).

9. Procédé in vitro selon l'une des revendications précédentes, **caractérisé en ce que** le profil diagnostique pour l'identification d'une cardiomyopathie induite par érythrovirus comprend en outre au moins 3 microARN ou acides nucléiques synthétiques ayant des séquences identiques ou complémentaires qui sont sélectionnées parmi le groupe composé des microARN suivants : hsa-miR-103b (SEQ ID N°: 117), hsa-miR-125a-5p (SEQ ID N°: 118), hsa-miR-1271-5p (SEQ ID N°: 119), hsa-miR-1274B (SEQ ID N°: 120), hsa-miR-138-1-3p (SEQ ID N°: 121), hsa-miR-146b-3p (SEQ ID N°: 122), hsa-miR-190a-5p (SEQ ID N° : 123), hsa-miR-199a-5p (SEQ ID N° : 124), hsa-miR-26b-3p (SEQ ID N° : 125), hsa-miR-320c (SEQ ID N° : 126), hsa-miR-3617-5p (SEQ ID N° : 127), hsa-miR-362-5p (SEQ ID N° : 128), hsa-miR-3663-3p (SEQ ID N°: 129), hsa-miR-367-3p (SEQ ID N°: 130), hsa-miR-3922-5p (SEQ ID N°: 131), hsa-miR-424-5p (SEQ ID N°: 132), hsa-miR-432-5p (SEQ ID N°: 133), hsa-miR-4429 (SEQ ID N°: 134), hsa-miR-4698 (SEQ ID N°: 135), hsa-miR-4726-3p (SEQ ID N° : 136), hsa-miR-4743-5p (SEQ ID N° : 137), hsa-miR-497-5p (SEQ ID N°: 138), hsa-miR-502-3p (SEQ ID N°: 139), hsa-miR-520d-3p (SEQ ID N°: 140), hsa-miR-520f-3p (SEQ ID N°: 141), hsa-miR-597-5p (SEQ ID N° : 142), hsa-miR-625-3p (SEQ ID N° : 143), hsa-miR-628-5p (SEQ ID N°: 144), hsa-miR-629-5p (SEQ ID N°: 145), hsa-miR-758-3p (SEQ ID N°: 146), mmu-miR-374-5p (SEQ ID N°: 147).

10. Procédé in vitro selon l'une des revendications précédentes, **caractérisé en ce que** le profil diagnostique pour l'identification d'une cardiomyopathie induite par ciHHV6 comprend en outre au moins 3 microARN ou acides nucléiques synthétiques ayant des séquences identiques ou complémentaires qui sont sélectionnées parmi le groupe composé des microARN suivants : hsa-let-7e-5p (SEQ ID N° : 148), hsa-miR-139-3p (SEQ ID N°: 149), hsa-miR-141-3p (SEQ ID N°: 150), hsa-miR-146b-3p (SEQ ID N°: 151), hsa-miR-181c-5p (SEQ ID N°: 152), hsa-miR-3156-5p (SEQ ID N°: 153), hsa-miR-362-5p (SEQ ID N°: 154), hsa-miR-411-5p (SEQ ID N°: 155), hsa-mir-422a (SEQ ID N° : 156), hsa-miR-4535 (SEQ ID N° : 157), hsa-miR-485-3p (SEQ ID N° : 158), hsa-miR-486-3p (SEQ ID N° : 159), hsa-miR-487b-3p (SEQ ID N°: 160), hsa-miR-494-3p (SEQ ID N°: 161), hsa-miR-511-5p (SEQ ID N°: 162), hsa-miR-520f-3p (SEQ ID N° : 163), hsa-miR-548c-3p (SEQ ID N° : 164), hsa-miR-548c-5p (SEQ ID N°: 165), hsa-miR-590-5p (SEQ ID N°: 166), hsa-miR-671-3p (SEQ ID N°: 167), hsa-miR-886-3p (SEQ ID N°: 168), hsa-miR-889-3p (SEQ ID N° : 169), hsa-miR-92a-3p (SEQ ID N° : 170).

11. Procédé in vitro selon l'une des revendications précédentes, **caractérisé en ce que** le profil diagnostique pour l'identification d'une cardiomyopathie induite par HHV6 comprend en outre au moins 3 microARN ou acides nucléiques synthétiques ayant des séquences identiques ou complémentaires qui sont sélectionnées parmi le groupe composé des microARN suivants : hsa-miR-192-5p (SEQ ID N°: 171), hsa-miR-29a-3p (SEQ ID N°: 172), hsa-miR-30c-5p (SEQ ID N°: 173), hsa-miR-483-5p (SEQ ID N°: 174), mmu-miR-374-5p (SEQ ID N° : 175).

12. Procédé in vitro selon l'une des revendications précédentes, **caractérisé en ce que** les étapes suivantes sont réalisées :
- fourniture d'un échantillon prélevé sur un patient chez lequel on suspecte la présence d'une cardiomyopathie non ischémique ou d'une maladie de surcharge du coeur,
- mise en contact de l'échantillon avec au moins deux sondes présentant chacune une séquence correspondant à ou complémentaire aux séquences des microARN parmi au moins un des groupes selon l'une des revendications 1 à 11, dans des conditions permettant une hybridation entre les microARN contenus dans l'échantillon et les sondes,
- détermination d'une hybridation réussie entre des microARN de l'échantillon et l'au moins une sonde, et
- détermination de la présence d'un microARN dans l'échantillon à l'aide du résultat de l'hybridation de l'étape précédente.
